# EUROPÄISCHE PATENTSCHRIFT

(11) **EP 0 565 553 B1**
(45) Veröffentlichungstag und Bekanntmachung des Hinweises auf die Patenterteilung: **21.07.1999**
(21) Anmeldenummer: 92901410.8
(22) Anmeldetag: 21.12.1991
(51) Int. Cl.: C07J 1/00, A61K 31/565, A61K 31/58, C07J 41/00, C07J 43/00, C07J 31/00, C07J 17/00, C07J 71/00

(54) **6,7-MODIFIZIERTE 11$g(b)-ARYL-4-ESTRENE**
6,7-MODIFIED 11$g(b)-ARYL-4-ESTRENES
11$g(b)-ARYL-4- STRENES MODIFIES EN POSITION 6,7

(30) Priorität: 22.12.1990 DE 4042007
(43) Veröffentlichungstag der Anmeldung: 20.10.1993
(73) Patentinhaber: SCHERING AKTIENGESELLSCHAFT, 13342 Berlin (DE)
(72) Erfinder: OTTOW, Eckhard, D-1000 Berlin 45 (DE); SCHWEDE, Wolfgang, D-1000 Berlin 10 (DE); CLEVE, Arwed, D-1000 Berlin 42 (DE); ELGER, Walter, D-1000 Berlin 33 (DE); CHWALISZ, Krzysztof, D-1000 Berlin 45 (DE); SCHNEIDER, Martin, D-1000 Berlin 28 (DE); FUHRMANN, Ulrike, D-1000 Berlin 15 (DE)
(86) Internationale Anmeldenummer: EP9102493
(87) Internationale Veröffentlichungsnummer: WO9211277

(56) Entgegenhaltungen:
- EP-A- 0 190 759
- EP-A- 0 277 089
- EP-A- 0 404 283
- DE-A- 4 018 167
- DE-A- 4 018 168

## Beschreibung

Die vorliegende Erfindung betrifft Verbindungen der allgemeinen Formel I 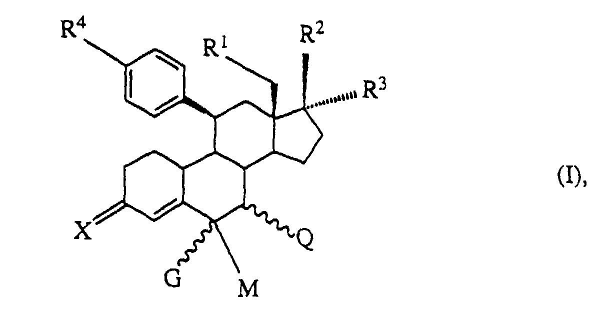 worin
- X: für ein Sauerstoffatom oder die Hydroxyiminogruppierung >N∼OH
- R¹: für ein Wasserstoffatom oder eine Methylgruppe,
- R²: für eine Hydroxygruppe, eine C₁-C₁₀-Alkoxy- oder C₁-C₁₀-Acyloxygruppe,
- R³: für ein Wasserstoffatom, die Gruppierung -(CH₂)ₙCH₂Z, wobei n 0, 1, 2, 3, 4 oder 5 ist, Z ein Wasserstoffatom, die Cyanogruppe oder den Rest -OR⁵ mit R⁵=H, C₁-C₁₀-Alkyl oder C₁-C₁₀-Acyl bedeuten, die Gruppierung -(CH₂)ₘC≡C-Y, wobei m 0, 1 oder 2 und Y ein Wasserstoff-, Fluor-, Chlor- Brom- oder Jod-Atom, einen C₁-C₁₀-Alkyl-, C₁-C₁₀-Hydroxyalkyl-, C₁-C₁₀-Alkoxyalkyl-, C₁-C₁₀-Acyloxyalkylrest bedeuten, die Gruppierung -(CH₂)ₚ-CH=CH-(CH₂)ₖCH₂R⁶, wobei p 0 oder 1 und k 0, 1 oder 2 und R⁶ ein Wasserstoffatom, eine Hydroxygruppe, einen C₁-C₄-Alkoxy- oder C₁-C₄-Acyloxyrest bedeuten,
oder aber R² und R³ gemeinsam für einen Rest der Formel 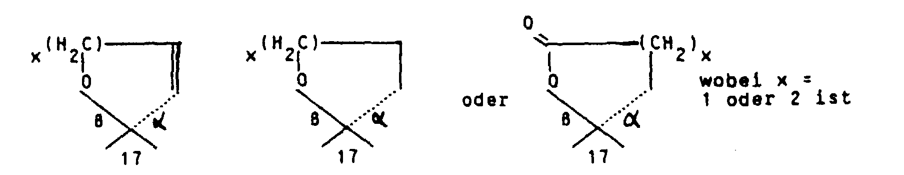
- R⁴: für ein Wasserstoffatom, eine Cyanogruppe, ein Chlor-, Fluor-. Brom-, Jodatom, für eine Trialkylsilyl-, Trialkylstannylgruppe, für einen geradkettigen oder verzweigten, gesättigten oder ungesättigten C₁-C₈-Kohlenwasserstoff-Acyl- oder Alkoxyalkylrest, für eine Aminogruppe 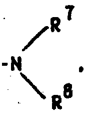 in welcher R⁷ und R⁸ unabhängig voneinander ein Wasserstoffatom oder eine C₁-C₄-Alkylgruppe bedeuten, oder für ein entsprechendes Aminoxid 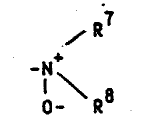 oder für die Gruppierungen -OR⁹ oder -S(O)ᵢR⁹ mit i = 0, 1 oder 2, in welchen R⁹ ein Wasserstoffatom, eine Methyl-, Ethyl-, Propyl-, Isopropyl-, Methoxyphenyl-, Allyl- oder eine 2-Dimethylaminoethylgruppe bedeuten, oder für einen Heteroarylrest der Formel Iα 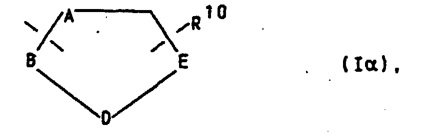 in welchem A ein Stickstoff-, Sauerstoff- oder Schwefelatom, -B-D-E- die Elementenfolge -C-C-C-, -N-C-C- oder -C-N-C- und R¹⁰ ein Wasserstoffatom, eine Cyanogruppe, ein Chlor-, Fluor-, Brom-, Jodatom, eine Trialkylsilyl-, Trialkylstannylgruppe, einen geradkettigen oder verzweigten, gesättigten oder ungesättigten C₁-C₈-Alkyl-, -Acyl- oder Alkoxyalkylrest, für eine Aminogruppe 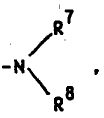 in welcher R⁷ und R⁸ unabhängig voneinander ein Wasserstoffatom oder eine C₁-C₄-Alkylgruppe bedeuten, oder ein entsprechendes Aminoxid 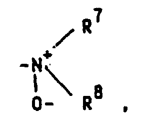 oder die Gruppierung -OR⁹ oder -S(O)ᵢR⁹ mit i = 0, 1 oder 2, in welchen R⁹ ein Wasserstoffatom, eine Methyl-, Ethyl-, Propyl-, Isopropyl-, Methoxyphenyl-, Allyl- oder eine 2-Dimethylaminoethylgruppe bedeuten, symbolisieren,
oder für einen Heteroarylrest der Formel Iβ 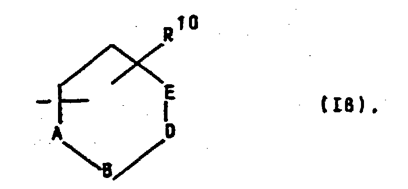 in welchem A ein Stickstoffatom und -B-D-E- die Elementenfolge -C-C-C-, -N-C-C-, -C-N-C- oder -C-C-N- bedeuten und R¹⁰ die bereits angegebene Bedeutung hat,
oder für einen Phenylrest der Formel Iγ 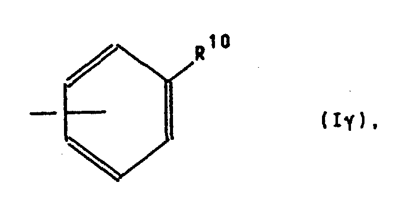 worin R¹⁰ die bereits angegebene Bedeutung hat,
G, wenn M und Q Wasserstoffatome darstellen, für ein Halogenatom oder einen C₁-C₄-Alkylrest oder wenn M und Q eine gemeinsame zusätzliche Bindung darstellen, für ein Wasserstoff-, ein Halogenatom oder einen C₁-C₄-Alkylrest, Q, wenn M und G Wasserstoffatome darstellen, für einen C₁-C₄-Alkylrest, wobei, wenn M und Q oder M und G Wasserstoffatome darstellen, R₄ nicht für eine Acylgruppe stehen kann, G und M gemeinsam, wenn Q ein Wasserstoffatom darstellt, für eine Methylen- oder Ethylengruppe,
stehen,
sowie deren pharmakologisch verträgliche Additionssalze mit Säuren, Verfahren zu ihrer Herstellung, diese Verbindungen enthaltende pharmazeutische Präparate, ihre Verwendung zur Herstellung von Arzneimitteln sowie die dazu benötigten neuen Zwischenprodukte.

Die Erfindung betrifft insbesondere Verbindungen, in denen X für ein Sauerstoffatom steht.

Die in R², R³, R⁵ und Y der allgemeinen Formel I enthaltenen Alkoxy-, Acyloxy-, Alkyl-, Acyl- sowie Hydroxyalkylgruppen sollen jeweils 1 bis 10 und die Alkoxyalkyl- oder Acyloxyalkylgrukppen in Y 2 bis 10 Kohlenstoffatome enthalten. Dabei sind als bevorzugte Gruppen von den Alkoxygruppen die Methoxy-, Ethoxy-, Propoxy- und Isopropoxygruppe zu nennen; von den Acyl(oxy)gruppen kommt der Formyl(oxyl-, Acetyl(oxy)- und Propionyl(oxy)gruppe besondere Bedeutung zu.

Bei den Alkylgruppen sind vor allem die Methyl-, Ethyl-, Propyl-, Isopropyl- sowie tert.-Butylgruppe zu nennen, und von den Hydroxyalkylgruppen sind die entsprechenden, in beliebiger Stellung mit einer Hydroxygruppe substituierten Reste bevorzugt.

Für n kommt insbesondere 0, 1, 2 und 3 infrage; wenn Z=CN, ist eine Cyanomethylgruppe (n=O) besonders bevorzugt. Außer den bereits genannten Gruppen kann Y vorzugsweise auch ein Wasserstoff-, Chlor- oder Bromatom sein.

Von den Alkenylresten in R³ sind die Propenyl- und Butenylgruppen, die in der E- oder Z-Konfiguration vorliegen können, bevorzugt, das heißt, wenn R³ für -(CH₂)ₚ-CH=CH-(CH₂)ₖ-CH₂-R⁶ steht, dann soll k vorzugsweise 0 oder 1 und p=O sein.

Unter den für R⁶ genannten Alkoxy- oder Acyloxygruppen, die sowohl geradkettig als auch verzweigt sein können, sind die Methoxy- Ethoxy-, Propoxy-, Isopropoxy- bzw. die Formyloxy-, Acetyloxy- und Propionyloxygruppe besonders bevorzugt.

Von den C₁-C₈ Kohlenwasserstoff- und Alkoxyalkylresten, die für R⁴ stehen können, sind dies vor allem der Methyl-, Ethyl-, Propyl-, Isopropyl-, Cyclopentyl- und Cyclohexylrest bzw. die Alkoxymethyl- bzw. 1- oder 2-Alkoxyethylgruppen mit den genannten Kohlenwasserstoffresten; von den C₁-C₈-Acylresten für R⁴ ist insbesondere an den Acetyl-, Propionyl- und Isobutyrylrest gedacht.

Steht R⁴ für die Aminogruppe 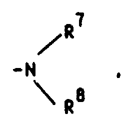 bedeuten R⁷ und R⁸ vorzugsweise jeweils einen Methylrest, doch auch dem Ethylrest kommt besondere Bedeutung zu, wobei dann entweder beide Reste am Stickstoffatom für einen Ethylrest oder einer für einen Methyl- und einer für einen Ethylrest stehen.

Für den Substituenten R⁹ sind die Methyl-, Ethyl- und 2-(Dimethylamino)ethylgruppe besonders hervorzuheben.

Von den gemäß Formel Iα möglichen Heteroarylresten sind der 3-Thienyl-, 3-Furyl- und 3-Pyrrolylrest bevorzugt mit R¹⁰ in der Bedeutung einer Cyano-, Methoxy- oder Dimethylaminogruppe.

Als Heteroarylreste der Formel Iβ kommen erfindungsgemäß insbesondere der 3- oder 4-Pyridyl-, der 5-Pyrimidinyl-, 4-Pyridazinyl- oder Pyrazinylrest infrage. Der Phenylrest der Formel Iγ weist als Substituenten R¹⁰ insbesondere die Cyano-, Methoxy- oder Dimethylaminogruppe auf, wobei sich wiederum diese Substituenten bevorzugt in der p-Position des Phenylringes befinden.

Für G soll als Halogenatom insbesondere das Chloratom und als C₁-C₄-Alkylrest die Methylgruppe bevorzugt sein.

Für Q soll als C₁-C₄-Alkylrest die Methyl- und Ethylgruppe bevorzugt sein.

G und M sollen gemeinsam bevorzugt für eine Methylengruppe stehen.

Nachstehend genannte Verbindungen sind erfindungsgemäß besonders bevorzugt:
17β-Hydroxy-11β-(4-methoxyphenyl)-17α-(prop-1-inyl)-4,6-estradien-3-on
17β-Hydroxy-11β-(4-methoxyphenyl)-7β-methyl-17α-(prop-1-inyl)-4-estren-3-on
17β-Hydroxy-11β-(4-methoxyphenyl)-7α-methyl-17α-(prop-1-inyl)-4-estren-3-on
17β-Hydroxy-17α-(3-hydroxyprop-1-(Z)-enyl)-6β-methyl-11β-[4-(3-pyridinyl)phenyl)-4-estren-3-on
17β-Hydroxy-17α-(3-hydroxyprop-1-(Z)-enyl)-6α-methyl-11β-[4-(3-pyridinyl)phenyl]-4-estren-3-on
17β-Hydroxy-17α-(3-hydroxyprop-1-inyl)-6β-methyl-11β-[4-(3-pyridinyl)phenyl]-4-estren-3-on
11β-(4-Dimethylaminophenyl)-17ß-hydroxy-17α-(prop-1-inyl)-4,6-estradien-3-on
11β-(4-Dimethylaminophenyl)-17α-ethinyl-17β-hydroxy-4,6-estradien-3-on
11β-[4-(4-Cyanphenyl)-phenyl]-17β-hydroxy-17α-(prop-1-inyl)-4,6-estradien-3-on
11β-[4-(4-Cyanphenyl)-phenyl)-17β-hydroxy-17α-(3-hydroxyprop-1-inyl)-4,6-estradien-3-on
11β-[4-(4-Cyanphenyl)-phenyl]-17β-hydroxy-17α-(3-hydroxyprop-1(Z)-enyl)-4,6-estradien-3-on
17β-Hydroxy-11β-[4-(4-methylthiophenyl)-phenyl]-17α-(prop-1-inyl)-4,6-estradien-3-on
17β-Hydroxy-11β-[4-(4-methylsulfinylphenyl)-phenyl]-17α-(prop-1-inyl)-4,6-estradien-3-on
17β-Hydroxy-11β-[4-(4-methylsulfonylphenyl)-phenyl]-17α-(prop-1-inyl)-4,6-estradien-3-on
11β-[4-(4-Acetylphenyl)-phenyl]-17β-hydroxy-17α-(3-hydroxyprop-1(Z)-enyl)-4,6-estradien-3-on
17β-Hydroxy-11β-[4-(3-furanyl]-phenyl]-6β-methyl-17α-(3-hydroxyprop-1(Z)-enyl-4-estren-3-on
17β-Hydroxy-11β-[4-(3-furanyl)-phenyl]-6α-methyl-17α-(3-hydroxyprop-1(Z)-enyl-4-estren-3-on
17β-Hydroxy-6β-methyl-17α-(3-hydroxyprop-1(Z)-enyl)-11β-[4-(3-thienyl)-phenyl]-4-estren-3-on
17β-Hydroxy-6α-methyl-17α-(3-hydroxyprop-1(Z)-enyl)-11β-[4-(3-thienyl)-phenyl]-4-estren-3-on
17β-Hydroxy-6β-methyl-17α-(3-hydroxyprop-1(Z)-enyl)-11β-[4-(2-thiazolyl)-phenyl]-4-estren-3-on
17β-Hydroxy-6α-methyl-17α-(3-hydroxyprop-1(Z)-enyl)-11β-[4-(2-thiazolyl)-phenyl]-4-estren-3-on
17β-Hydroxy-11β-[4-(5-pyrimidinyl)-phenyl]-6β-methyl-17α-(3-hydroxyprop1(Z)-enyl)-4-estren-3-on
17β-Hydroxy-11β-[4-(5-pyrimidinyl)-phenyl]-6α-methyl-17α-(3-hydroxyprop-1(Z)-enyl)-4-estren-3-on
17β-Hydroxy-11β-[4-(5-pyrimidinyl)-phenyl]-6β-methyl-17α-(prop-1-inyl)-4-estren-3-on

Bei den Verbindungen der allgemeinen Formel I handelt es sich um kompetitive Antagonisten des Progesterons (Antigestagene). Alle bis vor kurzem bekannt gewordenen steroidalen Antigestagene weisen neben einem Δ⁴Δ⁹-3-Oxo-chromophor einen vorzugsweise substituierten 11β-Phenylrest auf [A. Belanger, D. Philibert und G. Teutsch, Steroids 37, 2742 (1981); D. Philibert, T. Ojasoo und J.P. Raynand, Endocrinology 10, 1850 (1977), EP-A 057 115; G. Teutsch, T. Ojasoo und J.P. Raynand, J. Steroid Biochem. 31, 549 (1988)].

Als den erfindungsgemäßen Verbindungen nächstkommende bekannte Verbindungen sind die in der EP-A-0 190 759 beschriebenen 11β-(4-Acylphenyl)-4,9-estradien-3-one, die in 6- und/oder 7-Stellung α- oder β-ständig ein Halogenatom oder eine C₁-C₄-Alkylgruppe tragen können, zu nennen. Das 17-Kohlenstoffatom ist dabei wie für Antigestagene üblich substituiert.

Neuerdings sind auch Antigestagene steroidalen Ursprungs gefunden worden, in denen anstelle der 9,10-Doppelbindung eine Methylenbrücke zwischem dem 9-C-Atom und einem der ortho-C-Atome des 11β-Arylringes getreten ist (EP-A 0283428). Offensichtlich bewirkt die Einführung des 11β-Arylrestes den Übergang von gestagener zu antigestagener Wirksamkeit. Allerdings ist es bisher nicht gelungen, ein dem Progesteron am nächsten kommendes Antigestagen, sozusagen "das Antiprogesteron", welches keine 9,10-Doppelbindung, sondern neben einem 11β-Arylrest einen "freien" 10β-Substituenten, z.B. ein Wasserstoffatom, aufweisen würde, herzustellen. Versuche, 11β-[4-(Substituent)-aryl]-17β-hydroxy-5(10)-estren-3-on durch kurzzeitige Behandlung mit verdünnten Mineralsäuren in die entsprechende Verbindung mit einer 4(5)-Doppelbindung zu isomerisieren (Bedingungen, unter denen in der 11-unsubstituierten Reihe eine Doppelbindungsverschiebung von der 5(10)- in die 4(5)-Stellung ohne weiteres erfolgt), schlugen fehl [G. Neef, G. Sauer und R. Wiechert, Tetr. Lett., 24, 5205 (1983)].

Erstmals wurden 11β-4-Aryl-estrene in der deutschen Patentanmeldung P 39 21 059.6 beschrieben.

Die Herstellung der erfindungsgemäßen Verbindungen der allgemeinen Formel I ist in dem nachstehenden Reaktionsschema wiedergegeben:

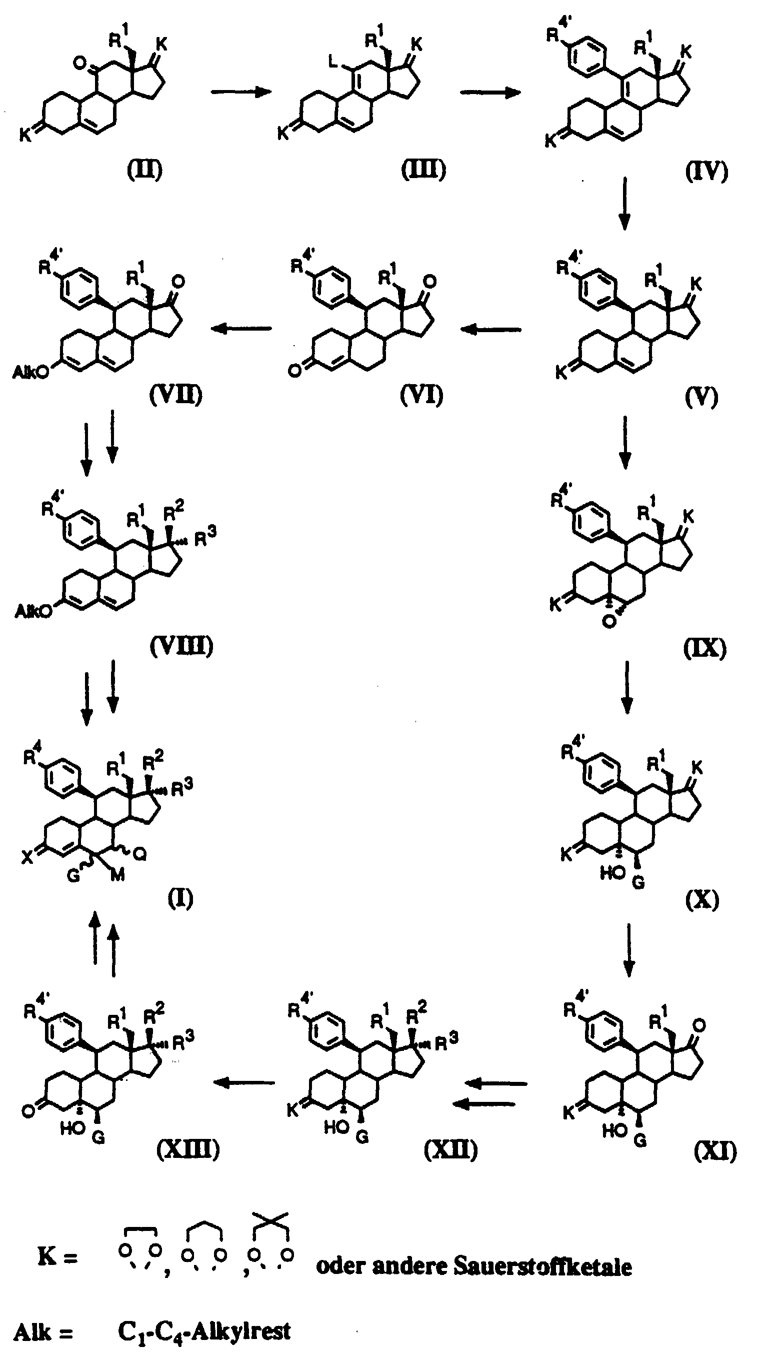

Gemäß der vorliegenden Erfindung wird Verbindung II [Recl. Trav. Chim. Bays-Pas 107, 331, (1988)] zunächst in eine Verbindung der Formel III überführt, wobei L für eine Perfluoralkylsulfonyloxygruppe CₙF₂ₙ₊₁SO₂O- (n=1,2,3,4) steht.

Verbindung III wird in Gegenwart einer katalytischen Menge eines Übergangsmetallkatalysators mit einer Arylverbindung der allgemeinen Formel Z 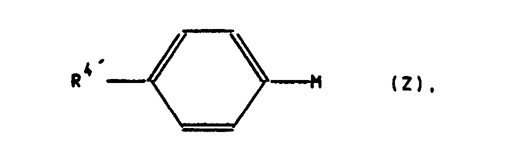 worin M für einen der Reste 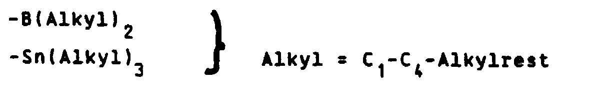 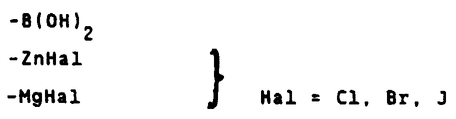 und R⁴, für einen der unter R⁴ genannten Reste stehen,
zu einer Verbindung der allgemeinen Formel IV,
worin R¹ die in Formel I und R⁴, die in Formel Z angegebene Bedeutung haben und gegebenenfalls, wenn R⁴ in der Formel I eine andere Bedeutung als R^{4'} in der Formel IV haben soll, eine Verbindung der allgemeinen Formel IV, worin R^{4'} für ein Bromatom steht oder nach Überführung einer für R^{4'} stehenden Methoxygruppe in eine Perfluoralkylsulfonyloxygruppe CₙF₂ₙ₊₁SO₂O-(n=1,2,3,4), mit einer Verbindung der allgemeinen Formel W

R⁴-M (W),

worin R⁴ die letztlich für diesen Substituenten in der Formel I gewünschte und M die bereits in Formel Z angegebene Bedeutung haben, umgesetzt.

Für L in der Verbindung III steht vorzugsweise die Trifluormethylsulfonyloxygruppe. Als Übergangsmetallkatalysator zur Kupplung der Arylverbindung der allgemeinen Formel Z mit der die Abgangsgruppe L aufweisenden Verbindung dient gemäß den Beispielen vorliegender Erfindung Palladiumtetrakistriphenylphosphin (siehe nachstehend angegebene Literatur); genausogut könnte aber Nickeltetrakistriphenylphosphin oder ähnliche Übergangsmetallkatalysatoren verwendet werden.

Die Variante, daß der letztendlich gewünschte Substituent R⁴ über die Funktionalisierung eines Brom- oder Methoxysubstituenten R^{4'} in der Verbindung IV eingeführt wird, ist dann zu wählen, wenn die zu kuppelnde Arylverbindung der allgemeinen Formel Z, worin R^{4'} bereits mit R⁴ identisch ist, nicht zugänglich oder zur Kupplung nicht geeignet ist. Übergangsmetallkatalysierte Arylkupplungsreaktionen von Verbindungen des Typs der allgemeinen Formel Z mit Verbindungen, die eine Abgangsgruppe tragen, sind beispielsweise beschrieben in: mit -Sn(Alkyl)₃-substituierten Aromaten: J.E. McMurry and S. Mohanraj, Tetrahydron Letters, 24, No. 27, S. 2723-2726, (1983); X. Lu und J. Zhu, Communications, S. 726-727. (1987); Q.-Y. Chen und Z.-Y. Yang, Tetrahedron Letters 27, No. 10, S. 1171-1174. (1986); S. Cacchi, P.G. Ciattini, E. Morera und G. Ortar, Tetrahedron Letters 27, No. 33, S. 3931-3934, (1986); A.M. Echavarren und J.K. Stille. J. Am. Chem. Soc. (1987), 109, S. 5478-5486 und J. Am. Chem. Soc. (1988). 110, S. 1557; mit -B(OH)₂ und -B(OAlkyl)₂-substituierten Aromaten: Y. Hoshino, N. Miyaura und A. Suzuki, Bull. Chem. Soc. Jpn. 61, 3008 (1988); H. Matsubasa, K. Seto, T. Tahara und S. Takahashi; Bull. Chem. Soc. Jpn. 62, 3896 (1989); mit -ZnCl-substituierten Aromaten: R. McCague, Tetr. Lett., 28, 701 (1987); A. Arcadi, A. Burini. S. Cacchi, M. Delmastro, F. Marinelli. B. Pietroni, Syn. Les., 1, (1990), S. 47.

Die Verbindungen der allgemeinen Formel V, die als Ausgangsprodukte für die Herstellung der 10β-H-Steroide der allgemeinen Formel I geeignet sind, lassen sich bequem herstellen, indem eine Verbindung der Formel IV, worin R^{4'} und R¹ die in den Formeln angegebene Bedeutung haben, ohne Zerstörung des aromatischen Systems und der 5,6-Doppelbindung zu einer Verbindung der allgemeinen Formel V, worin R⁴ und R¹ die bereits angegebene Bedeutung haben, reduziert wird.

Bei der Reduktion von IV bildet sich die 11β-Arylverbindung V (stereoselektive Reduktion).

Zur Reduktion der 9(11)-Doppelbindung in IV kommen erfindungsgemäß verschiedene Methoden infrage: Erfindungsgemäß bevorzugt ist die Reduktion mit einem elektropositiven Metall in einem elektronensolvatisierenden Lösungsmittel oder in einem einen Lösungsvermittler enthaltenden Lösungsmittel. Als elektronensolvatisierendes Lösungsmittel kommt in erster Linie Ammoniak inbetracht.

Für die Reduktion genügen bereits equimolare Mengen Reduktionsmittel, es kann jedoch auch ein beträchtlicher Überschuß Reduktionsmittel verwendet werden, ohne daß das aromatische System und/oder die 5,6-Doppelbindung angegriffen werden/wird.

Als elektropositive Metalle sind alle für eine Birch-Reduktion geeigneten Metalle verwendbar. Erfindungsgemäß sind Lithium, Kalium, Natrium und Calcium - und von diesen Lithium insbesondere - bevorzugt.

Die Verbindungen der allgemeinen Formel V können nun auf unterschiedliche Art und Weise, je nach letztendlich gewünschter Endverbindung der allgemeinen Formel I, weiterverarbeitet werden.

Einerseits kann die Diketalverbindung V durch Behandlung mit einer starken Säure in das entsprechende 3,17-Diketon der allgemeinen Formel VI überführt werden.

Aus diesem wird anschließend nach gängigen Methoden (siehe experimenteller Teil) ein Dienolether der allgemeinen Formel VII (Alk = C₁- bis C₄-Alkylrest) hergestellt.

Der Dienolether VII dient als Substrat zur Einführung der am C-17-Atom gewünschten Substituenten R² und R³.

Diese Einführung erfolgt analog literaturbekannten Verfahren (z.B. J. Fried, J.A. Edwards, "Organic Reactions in Steroid Chemistry", Van Nostrand Reinhold Company, 1972, Vol. 1 und 2; Terpenoids and Steroids", Specialist Periodical Report, The Chemical Society, London, Vol. 1-2) durch nucleophile Addition an das C-17-Keton.

Die Einführung des Substituenten -C≡C-Y als R³, wobei Y die oben angegebene Bedeutung hat, erfolgt mit Hilfe einer metallierten Verbindung der allgemeinen Formel MC≡C-Y', in der Y' eine Alkin-Schutzgruppe, wie zum Beispiel Trimethylsilyl oder tert.-Butyldimethylsilyl, ist.

Die metallorganische Verbindung kann auch in situ gebildet und mit dem 17-Keton zur Reaktion gebracht werden. So kann man zum Beispiel auf das 17-Keton in einem geeigneten Lösungsmittel Acetylen und ein Alkalimetall, insbesondere Kalium, Natrium oder Lithium, in Gegenwart eines Alkohols oder in Gegenwart von Ammoniak einwirken lassen. Das Alkalimetall kann auch in Form von zum Beispiel Methyl- oder Butyllithium zur Einwirkung kommen. Als Lösungsmittel sind insbesondere Dialkylether, Tetrahydrofuran, Dioxan, Benzol und Toluol geeignet.

Die Einführung von 3-Hydroxypropin, -propen in 17-Stellung erfolgt durch Umsetzung des 17-Ketons mit dem Dianion des Propargylalkohols (3-Hydroxypropin), zum Beispiel dem in situ generierten Dikaliumsalz des Propargylalkohols, zum 17α-(3-Hydroxyprop-1-inyl)-17β-hydroxyderivat oder mit metallierten Derivaten des 3-Hydroxypropins, zum Beispiel mit 1-Lithium-3-(tetrahydropyran-2'-yloxy)-prop-1-in-1-id, zum 17-[3-(Tetrahydropyran-2'-yloxy)-prop-1-inyl]-17β-hydroxyderivat, die anschließend zu den 17-(3-Hydroxypropyl- bzw. Hydroxypropenyl)17β-hydroxy-Verbindungen hydriert werden können. Das gelingt zum Beispiel durch Hydrierung bei Raumtemperatur und Normaldruck in Lösungsmitteln wie Methanol, Ethanol, Propanol, Tetrahydrofuran (THF) oder Essigester unter Zusatz von Edelmetall-Katalysatoren wie Platin oder Palladium.

Die Einführung homologer Hydroxyalkin-, Hydroxyalken- und Hydroxyalkangruppen erfolgt in entsprechender Weise mit Homologen des Propargylalkohols.

Die Verbindung mit der Z-konfigurierten Doppelbindung in der Hydroxypropenylgruppe entsteht durch Hydrieren der acetylenischen Dreifachbindung mit einem desaktivierten Edelmetallkatalysator [J. Fried, J.A. Edwards: Organic Reactions in Steroid Chemistry, Van Nostrand Reinhold Company (1972), Seite 134; und H.O. House: Modern Synthetic Reactions {1972), Seite 19]. Als desaktivierte Edelmetallkatalysatoren kommen beispielsweise 10 % Palladium auf Bariumsulfat in Gegenwart eines Amins oder 5 % Palladium auf Calciumcarbonat unter Zusatz von Blei(II)acetat infrage. Die Hydrierung wird nach der Aufnahme von einem Äquivalent Wasserstoff abgebrochen.

Die Verbindung mit der E-konfigurierten Doppelbindung in der Hydroxypropenylgruppe entsteht durch Reduktion der acetylenischen Dreifachbindung in an sich bekannter Weise. In der Literatur sind eine ganze Reihe von Methoden zur Umwandlung von Alkinen in trans-Olefine beschrieben, beispielsweise die Reduktion mit Natrium in flüssigem Ammoniak [J. Am. Chem. Soc. 63 (1941) 216], mit Natriumamid in flüssigem Ammoniak [J.A. Chem. Soc. (1955) 3558], mit Lithium in niedermolekularen Aminen (J.A. Chem. Soc. 77 (1955) 3378], mit Boranen (J.Am. Chem. Soc. 93 (1971) 3395 und 94 (1972) 6560], mit Diisobutylaluminiumhydrid und Methyl-Lithium (J. AM. Chem. Soc. 89 (1967) 5085] und insbesondere mit Lithiumaluminiumhydrid/Alkoholat (J. Am. Chem. Soc. 89 (1967) 4245]. Eine weitere Möglichkeit ist die Reduktion der Dreifachbindung mit Chrom(II)-sulfat in Gegenwart von Wasser oder Dimethylformamid in schwach saurem Milieu (J. Am. Chem. Soc. 86 (1964) 4358] sowie allgemein die Reduktion durch Einwirkung von Übergangsmetallverbindungen unter Wechsel der Oxydationsstufe.

Die Einführung der Hydroxyalkene kann auch direkt erfolgen durch Addition einer entsprechenden metallierten Hydroxyllkenylverbindung, wie zum Beispiel 1-Lithium-3-(tetrahydropyran-2'-yloxy)-prop-1(E)-en [J. Org. Chem. 40 2265) oder 1-Lithium-3-(tetrahydropyran-2'-yloxy)-prop-1(Z)-en (Synthesis 1981. 999]. Homologe können auf diese Art ebenfalls eingeführt werden.

Die Einführung von 3-Hydroxypropan in 17-Stellung kann ebenfalls direkt durch Umsetzung des 17-Ketons mit metallierten Derivaten von 3-Halogen-propanolen - wobei die Hydroxygruppe im Metallierungsschritt als Alkoholat [Tetrahedron Letters (1978), 3013] oder als geschützte Funktion [J. Org. Chem. 37, (1947)] vorliegt - zu der 17-(3-Hydroxypropyl)-17β-hydroxy-Verbindung bzw. zu der an der terminalen Hydroxygruppe geschützten Verbindung erfolgen. Als Schutzgruppen kommen zum Beispiel die Ethoxyethyl-, Tetrahydropyranyl- und Methoxymethylgruppen infrage.

Werden Endprodukte der Formel I gewünscht mit R²/R³ in der Bedeutung von 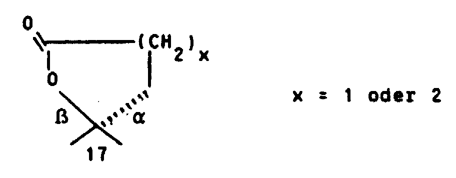 so wird die 17-(3-Hydroxypropyl)- bzw. 17-(4-Hydroxybutyl)-Verbindung in an sich bekannter Weise oxydiert, zum Beispiel mit Jones' Reagenz, Braunstein, Pyridiniumdichromat, Pyridiniumchlorochromat, Chromsäure-Pyridin oder dem Fetizon-Reagenz Silbercarbonat/Celite [Compt, rend. 267 (1968) 900].

Die Darstellung von Endprodukten der Formel I mit R²/R³ in der Bedeutung von 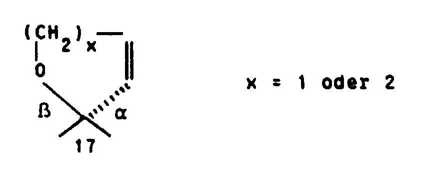 erfolgt durch Ringschlußreaktion des entsprechenden 17-(3-Hydroxyprop-1-(Z)-enyl- bzw. 17-(4-Hydroxybut-1-(Z)-enyl-17-β-hydroxy-Eduktes. Hydrierung des ungesättigten 5- oder 6-Ring-Spiroethers am Palladium/Aktivkohle-Kontakt führt zu den gesättigten Spiroethern.

Der Aufbau der 17-Cyanmethylseitenkette erfolgt in an sich bekannter Weise aus dem 17-Keton zum Beispiel über das 17-Spiroepoxid und Spaltung des Spiroepoxids mit HCN gemäß Z [Chem, 18 (1978) 259-260].

Auch die Einführung der 17-Hydroxyacetylseitenkette erfolgt nach an sich bekannten Methoden, beispielsweise nach den in J. Org. Chem. 47 (1982), 2993-2995, Chem. Ber. 113 (1984), 1184 bzw. US-Patent 4.600.538 beschriebenen Methoden.

Ausgehend von der die Seitenketten enthaltenden Verbindung der allgemeinen Formel VIII wird dann durch Dienoletherbromierung und anschließende Bromwasserstoffabspaltung [J. Fried, J.A. Edwards, Organic Reactions in Steroid Chemistry, Van Nostrand Reinhold Company 1972, S. 265-374; Tetrahedron 42, (1986) 2971] die 6,7-Doppelbindung neben der 3,4-Doppelbindung eingeführt.

Die Dienoletherbromierung wird zum Beispiel analog der Vorschrift in Steroids I, 233 durchgeführt.

Die Bromwasserstoffabspaltung unter Ausbildung der Δ⁶-Doppelbindung erfolgt durch Erhitzen der 6-Bromverbindung mit basischen Mitteln, vorzugsweise mit Lithiumbromid und Lithiumcarbonat oder mit Lithiumbromid und Calciumcarbonat in einem aprotischen Lösungsmittel wie Dimethylformamid bei Temperaturen von 50 bis 120 °C. Eine weitere Möglichkeit der HBr-Abspaltung besteht darin, daß man die 6-Bromverbindung in Collidin oder Lutidin erhitzt.

Die Einführung einer 6-Methylengruppe kann zum Beispiel ausgehend von einem 3-Amino-3(4),5(6)-dien-Derivat durch Umsetzung mit Formalin in alkoholischer Lösung [Helv. Chim. Acta. 56 (1973) 2396] zur 6α-Hydroxymethylgruppe und anschließender saurer Wasserabspaltung zum Beispiel mit Salzsäure in Dioxan/ Wasser oder ausgehend von einem 3-Alkoxy-3(4),5(6)-dien-Derivat, analog der im US-Patent 4,544,555 beschriebenen Methode oder direkt, ausgehend von einem 3-Oxo-4(5)-en-Derivat analog der Vorschrift in Synthesis (1982) 34 erfolgen.

Alkylierte Verbindungen in Position 7 werden durch 1,6-Addition an die entsprechenden Enone nach bekannten Methoden [J. Fried, J.A. Edwards: Organic Reactions in Steroid Chemistry, Van Nostrand Reinhold Company 1972, seiten 75-82, 2; und J. Am. Chem. Soc. 99 (1917) 1673] erhalten.

Andererseits ist es möglich, zur Weiterverarbeitung die Zwischenverbindungen der allgemeinen Formel V zu epoxidieren, zum Beispiel unter Verwendung organischer Persäure oder in Gegenwart von Hexachloraceton oder Nitrotrifluoracetophenon. Die gebildeten Epoxide der allgemeinen Formel IX lassen sich dann unter Verwendung komplexer Hydride oder einer Verbindung GMgHal (G = C₁- bis C₄-Alkylrest, Hal = Halogenatom) öffnen, wobei eine Verbindung der allgemeinen Formel X entsteht.

Der nächste Reaktionsschritt dient dem Aufbau des Substituenten R⁴ bzw. R^{4'} in p-Stellung am 11β-Phenylring.

Diese Vorgehensweise ist dann erforderlich, wenn R⁴ nicht direkt bei der Kupplung der Verbindung III mit der Arylverbindung Z zur Verbindung IV eingeführt wird.

Als Ausgangspunkt für diesen Aufbau dient die Verbindung X, worin R⁴ = OH ist, die aus der entsprechenden Methoxyverbindung durch Etherspaltung, beispielsweise mit Natriumethanthiolat in einem Lösungsmittel wie Dimethylformamid, erhältlich ist.

Durch Umsetzung der Hydroxyverbindung mit einem Perfluor-(C₁-C₄)-alkylsulfonsäureanhydrid oder -halogenid in Gegenwart einer Base wie Pyridin oder 4-(Dimethylamino)-pyridin gelangt man zur entsprechenden 11β-[4-(perfluoralkylsulfonyloxy)phenyl]-Verbindung [P.J. Stang, M. Hanack und L.R. Subramanian, Synthesis 85, (1982)].

Bei der sich anschließenden Kukpplung der 118-Arylverbindung mit R^{4"}-Sn(Alkyl)₃ oder R^{4"}-BL₂ wird entweder so vorgegangen, daß in einer übergangsmetallkatalysierten Reaktion (vorzugsweise Pd°) die Perfluoralkylsulfonatabgangsgruppe unter im wesentlichen fast gleichzeitiger Substitution durch den gewünschten Substituenten oder dessen Vorstufe verdrängt wird (Arylkupplungen mit Zinnverbindungen: J.E. McMurry and S. Mohanraj, Tetrahedron Letters 24, No. 27, S. 2723-2726, (1983); X. Lu und J. Zhu, Communications, S. 726-727, (1987); Q.-Y. Chen und Z.-Y. Yang, Tetrahedron Letters 27, No. 10, S. 1171-1174, (1986); S. Cacchi, P.G. Ciattini, E. Morera und G. Ortar, Tetrahedron Letters 27, No. 33, S. 3931-3934, (1986); A.M. Echavarren und J.K. Stille. J. Am. Chem. Soc. (1987), 109. S. 5478-5486; mit Borverbindungen: Synthesis 936 (1984), Chem. Pharm. Bull. 33, 4755-4763 (1985); J. Org. Chem. 49, 5237-52433 (1984); Bull. Chem. Soc. Jpn. 61, 3008-3010 (1988) oder es wird aus der Perfluoralkylsulfonat-Verbindung intermediär und übergangsmetallkatalysiert eine entsprechende Tri-organylstannyl-, vorzugsweise Tri-n-alkylstannyl-Verbindung hergestellt [J.K. Stille, Angew. Chem. 98 (1986), S. 504-519]. Diese wird anschließend in einer Eintopf-Reaktion mit einem halogen-, vorzugsweise brom- oder jodsubstituierten carbocyclischen oder heterocyclischen Aromaten [Y. Yamamoto, Y. Azuma, H. Mitoh, Communications, S. 564-565, (1986); T.J. Bailey. Tetrahedron Letters, 27, No. 37, S. 4407-4410, (1986)], der gegebenenfalls noch weitere Substituenten tragen kann, umgesetzt; der 11β-Phenylrest weist darin dann die gewünschte bzw. einen Vorläufer der gewünschten Substitution auf.

Zahlreiche derartige Umsetzungen mit Steroiden, in denen eine Trifluormethansulfonat-Gruppe sich in 4-Stellung eines 11β-Phenylringes befindet, sind in der EP-A-0283428 beschrieben.

Dann wird selektiv das 17-Ketal zum 17-Keton (allgemeine Formel XI) mit einer schwachen Säure (Oxalsäure) gespalten und die C-17-Seitenketten wie bereits vorstehend beschrieben aufgebaut. Die erwähnte selektive Ketalspaltung kann auch bereits auf der Triflatstufe erfolgen und das Triflat auf der 17-Ketonstufe weiterverarbeitet werden.

In der nach dem Seitenkettenaufbau erhaltenen Verbindung der allgemeinen Formel XII wird nun die 3-Ketoschutzgruppe unter milden sauren Bedingungen (Essigsäure oder 4N Salzsäure bei 0 °C) entfernt.

Durch Behandlung der gebildeten Verbindungen der allgemeinen Formel XIII mit verdünnter wäßriger Natronlauge werden die 3-Keto-4-en-Verbindungen der allgemeinen Formel I mit β-ständigem Substituenten G erhalten, während sich bei der Umsetzung von XIII mit wäßriger Salzsäure (oder einer anderen starken Säure) die entsprechenden Verbindungen der allgemeinen Formel I mit α-ständigem Substituenten G bilden.

Freie Hydroxygruppen können in an sich bekannter Weise alkyliert oder acyliert werden.

Gegebenenfalls kann auch zuerst der Substituent R⁴ aufgebaut werden und anschließend die Einführung der Substituenten R² und R³ vorgenommen werden, je nachdem, ob die Verfahrensbedingungen des zweiten Reaktionsschrittes die zuerst eingeführten oder aufgebauten Substituenten beeinträchtigen. Prinzipiell läßt sich der teilweise oder vollständige Aufbau des p-Substituenten am 11β-Phenylrest auf jeder Reaktionsstufe ab Verbindung IV durchführen.

Noch vorhandene Schutzgruppen werden nach gängigen Methoden abgespalten.

Die erhaltenen Verbindungen der allgemeinen Formel I mit X in der Bedeutung eines Sauerstoffatoms können gewünschtenfalls durch Umsetzung mit Hydroxylaminhydrochlorid in Gegenwart von tertiären Aminen bei Temperaturen zwischen -20 und +40 °C in die Oxime (Formel I mit X in der Bedeutung der Hydroxyiminogruppierung >N∼OH, wobei die Hydroxygruppe syn- oder antiständig sein kann) überführt werden. Geeignete tertiäre Basen sind beispielsweise Trimethylamin, Triäthylamin, Pyridin, N,N-Dimethylaminopyridin, 1,5-Diazabicyclo[4.3.0]non-5en (DBN) und 1,8-Diazabicyclo[5.4.0]undec-7-en (DBU), wobei Pyridin bevorzugt ist.

Die Entfernung der 3-Oxogruppe zu einem Endprodukt der allgemeinen Formel I mit X in der Bedeutung von 2 Wasserstoffatomen kann z.B. nach der in der DE-A-2805490 angegebenen Vorschrift durch reduktive Spaltung des Thioketals erfolgen.

Die neuen Verbindungen der allgemeinen Formel I sowie deren Additionssalze mit pharmazeutisch verträglichen Säuren sind wertvolle Pharmaka. So verfügen sie über eine starke Affinität zum Gestagenrezeptor und besitzen überraschend starke antigestagene sowie antiglucocorticoide, antimineralcorticoide und antiandrogene Eigenschaften. Diese wichtigen biologischen Wirksamkeiten können für medizinische Zwecke genutzt werden.

Wirkstoffe dieser Art mit ausgeprägter antigestagener Aktivität sind zur Auslösung von Aborten geeignet, da sie das zur Aufrechterhaltung der Schwangerschaft erforderliche Progesteron vom Rezeptor verdrängen. Sie sind deshalb wertvoll und interessant im Hinblick auf ihre Verwendung zur postcoitalen Fertilitätskontrolle.

Die neuen Verbindungen können außerdem zur Behandlung der Endometriose dienen. Sie können auch gegen hormonelle Unregelmäßigkeiten, zur Menstruationsauslösung und zur Geburtseinleitung eingesetzt werden. Außerdem können sie für die Behandlung von hormonabhängigen Carcinomen eingesetzt werden.

Die erfindungsgemäßen Verbindungen der allgemeinen Formel I sowie deren Additionssalze mit pharmazeutisch verträglichen Säuren weisen auch eine antiglucocorticoide Aktivität auf und können somit auch als Arzneimittel zur Therapie corticoid-induzierter Störungen (Glaukom) sowie zur Bekämpfung von Nebenwirkungen, die bei langfristiger Behandlung mit Glucocorticoiden auftreten (Cushing-Syndrom), eingesetzt werden. Sie ermöglichen daher auch die auf eine Supersekretion der Glucocorticoide zurückzuführenden Störungen, vor allem die Adipositas, Arteriosklerose, Hypertension, Osteoporose, Diabetes sowie die Insomnie zu bekämpfen.

Die erfindungsgemäßen Verbindungen der allgemeinen Formel I sowie deren Additionssalze mit pharmazeutisch verträglichen Säuren mit antiandrogener Aktivität können bei der Behandlung der Hypertrophie und des Prostatakarzinoms verwendet werden. Sie ermöglichen weiterhin eine spezifische Therapie von Androgenisierungserscheinungen bei Frauen: die pathologische Behaarung bei Hirsutismus, die androgenetische Alopezie sowie die gesteigerte Talgdrüsenfunktion bei Akne und Seborrhoe sind günstig beeinflußbar.

Die Erfindung betrifft somit auch Arzneimittel auf Basis der Verbindungen der allgemeinen Formel I sowie deren Additionssalze mit pharmazeutisch verträglichen Säuren, gegebenenfalls zusammen mit den üblichen Hilfs- und Trägerstoffen.

Die erfindungsgemäßen Verbindungen und deren Salze können nach an sich bekannten Methoden der Galenik zu pharmazeutischen Präparaten für die enterale, perkutane, parenterale oder lokale Applikation verarbeitet werden. Sie können in Form von Tabletten, Dragees, Gelkapseln, Granulaten, Suppositorien, Implantaten, injizierbaren sterilen wäßrigen oder öligen Lösungen, Suspensionen oder Emulsionen, Salben, Cremes und Gelen verabreicht werden.

Der oder die Wirkstoffe können dabei mit den in der Galenik üblichen Hilfsstoffen wie z.B. Gummiarabikum, Talk, Stärke, Mannit, Methylcellulose, Laktose, Tensiden wie Tweens^{(R)} oder Myrj^{(R)}, Magnesiumstearat, wäßrigen oder nichtwäßrigen Trägern, Paraffinderivaten, Netz-, Dispergier-, Emulgier-, Konservierungsmitteln und Aromastoffen zur Geschmackskorrektur (z.B. ätherischen Ölen) gemischt werden.

Die Erfindung betrifft somit auch pharmazeutische Zusammensetzungen, die als Wirkstoff zumindest eine erfindungsgemäße Verbindung oder eines ihrer Additionssalze mit pharmazeutisch verträglichen Säuren enthalten. Als Additionssalze der erfindungsgemäßen Produkte mit Säuren sind insbesondere die Hydrochloride und die Methansulfonate zu nennen. Eine Dosiseinheit enthält etwa 1 - 100 mg Wirkstoff(e).

Die Dosierung der erfindungsgemäßen Verbindungen liegt beim Menschen bei etwa 1 - 1000 mg pro Tag.

Die nachfolgenden Ausführungsbeispiele dienen der näheren Erläuterung der Erfindung:

### Beispiel 1

### 17β-Hydroxy-11β-(4-methoxyphenyl)-17α-(prop-1-inyl)-4,6-estradien-3 on

a) 3,3;17,17-Bis-(ethylendioxy)-11-trifluormethylsulfonyloxy-5,9(11)-estradien
26,1 g (69,7 mmol) 3,3;17,17-Bis-(ethylendioxy)-5-estren-11-on werden in 350 ml absolutem Methylenchlorid gelöst und unter Schutzgas mit 18 ml 2,6-Di-tertiärbutylpyridin versetzt. Nach Kühlen dieser Lösung auf 0°C werden 12,9 ml (76,8 mmol) Trifluormethansulfonsäureanhydrid langsam zugetropft. Danach wird das Reaktionsgemisch 20 Stunden bei Raumtemperatur nachgerührt. Zur Aufarbeitung wird es auf gesättigte Natriumhydrogencarbonatlösung gegossen, die organische Phase abgetrennt und die wäßrige mit Methylenchlorid nachextrahiert. Die vereinigten organischen Phasen werden mit gesättigter Natriumchloridlösung gewaschen, über Natriumsulfat getrocknet und am Vakuum eingeengt. Nach Chromatographie des Rohprodukts an Kieselgel mit einem Gemisch aus Etylacetat/Hexan werden neben 16,4 ml 2,6-Di-tertiär-butylpyridin und 5,1 g 3,3;17,17-Bis-(ethylendioxy)-5-estren-11-on 27 g 3,3;17,17-Bis-(ethylendioxy)-11-trifluormethylsulfonyloxy-5,9(11)-estradien als weißer Schaum erhalten.
[α]²⁰_{D} = + 104 ° (CHCl₃;c=0.505)
¹H-NMR (CDCl₃) δ [ppm]: 5,58 (1H,d breit J=5Hz,H-6); 3,7-4,0 (8H,m,H-Ketale); 2,88 (1H,d breit J=11Hz,H-10); 2,74 (1H,dtr J=16Hz und J=2,5Hz,H-12); 2,18-2,33 (2H,m, H-4); 0,84 (3H,s,H-18).
b) 3,3;17,17-Bis-(ethylendioxy)-11-(4-methoxyphenyl)-5,9(11)-estradien
**Methode 1:** 21,6 g (42,6 mmol) 3,3;17,17-Bis-(ethylendioxy)-11-trifluormethylsulfonyloxy-5,9(11)-estradien werden in einem Gemisch aus 360 ml Toluol und 170 ml Ethanol gelöst und nacheinander mit 2,5 g Palladiumtetrakistriphenylphosphin, 3,6 g Lithiumchlorid, 55 ml 2 m Natriumcarbonatlösung und 7,2 g (46,8 mmol) 4-Methoxyphenylboronsäure versetzt. Das Reaktionsgemisch wird dann 2 Stunden bei 95 °C gerührt, auf Raumtemperatur abgekühlt und mit gesättigter Natriumchloridlösung versetzt. Die organische Phase wird abgetrennt, nacheinander mit 5 %-iger Natronlauge und Wasser gewaschen, über Natriumsulfat getrocknet und am Vakuum eingeengt. Der Rückstand wird an Kieselgel mit einem Gemisch aus Ethylacetat/Hexan chromatographiert. Man erhält 19,2 g 3,3;17,17-Bis-(ethylendioxy)-11-(4-methoxyphenyl)-5,9(11)-estradien als weißen Schaum.
**Methode 2:** 1,52 g 3,3;17,17-Bis-(ethylendioxy)-11-trifluormethylsulfonyloxy-5,9(11)-estradien werden in 25 ml absolutem Dimethylformamid gelöst und mit 270 mg Lithiumchlorid und 350 mg Tetrakistriphenylphosphinpalladium versetzt. Nach fünfminütigem Nachrühren wird das Reaktionsgemisch mit 1,3 ml Tri-n-butyl-4-methoxyphenylzinn versetzt, 3 Stunden bei 110 °C unter Schutzgas gerührt, auf Rammtemperatur abgekühlt und mit Ethylacetat verdünnt. Nach Filtration über Celite und Waschen des Filterrückstandes mit Ethylacetat wird die organische Phase mit gesättigter Natriumchloridlösung gewaschen, über Natriumsulfat getrocknet und am Vakuum eingeengt. Chromatographie des Rückstandes an Aluminiumoxid (neutral, Stufe III) mit einem Gemisch aus Ethylacetat/Hexan ergeben 1,15 g 3,3;17,17-Bis-(ethylendioxy)-11-(4-methoxyphenyl)-5,9(11)-estradien als weißen Schaum.
Exemplarisch ist die Darstellung einiger weiterer Produkte analog den obigen Methoden 1) oder 2) in der nachstehenden Tabelle aufgeführt :

| **Aromat** | | **Produkt** | **Ausbeute [%]** | **Physikalische Daten** |
|---|---|---|---|---|
| a) | 4-Methoxyphenylboronsäure oder | 3,3;17,17-Bis-(ethylendioxy)-11-(4-methoxyphenyl)-5,9(11)-estradien | 97 | Fp. = 156 °C (Diisopropylether) [α]_{D}²⁰ = -0,1 ° (CHCl₃; c=0.52) |
| b) | Tri-n-butyl-4-methoxyphenylzinn | | 83 | |
| a) | 4-Methylphenylboronsäure | 3,3;17,17-Bis-(ethylendioxy)-11-(4-methylphenyl)-5,9(11)-estradien | 92 | Fp. = 175 °C (Diisopropylether) [α]_{D}²⁰ = -11° (CHCl₃; c=0.505) |
| a) | Phenylboronsäure | 3,3;17,17-Bis-(ethylendioxy)-11-phenyl-5,9(11)-estradien | | Fp. = 189 °C (Diisopropylether) [α]_{D}²⁰ = - 3° (CHCl₃; c=0.5) |
| a) | 4-Bromphenylboronsäure | 3,3;17,17-Bis-(ethylendioxy)-11-(4-bromphenyl)-5,9(11)-estradien | 62 | Fp. = 171°C (Diisopropylether) [α]_{D}²⁰ = - 15° (CHCl₃; c=0.5) |

c) 3,3;17,17-Bis-(ethylendioxy)-11β-(4-methoxyphenyl)-5-estren
800 ml Ammoniak werden bei -70 °C kondensiert und mit 1,39 g Lithium versetzt. Nach Auftreten der charakteristischen Blaufärbung werden 18,6 g (40 mmol) 3,3;17,17-Bis-(ethylendioxy)-11-(4-methoxyphenyl)-5,9(11)-estradien gelöst in 400 ml Tetrahydrofuran zugetropft. Nach 20-minütigem Nachrühren zersetzt man das überschüssige Lithium durch Zugabe von Wasser, dampft das Ammoniak ab, gießt das Reaktionsgemisch auf gesättigte Ammoniumchloridlösung und extrahiert die wäßrige Phase mit Ethylacetat. Die vereinigten organischen Phasen werden mit gesättigter Natriumchloridlösung gewaschen, über Natriumsulfat getrocknet und am Vakuum eingeengt. Nach Chromatographie des Rohprodukts an Kieselgel mit einem Gemisch aus Etylacetat/Hexan werden 15,2 g 3,3;17,17-Bis-(ethylendioxy)-11β-(4-methoxyphenyl)-5-estren und 1,4 g 17,17-Bis-(ethylendioxy)-11-(4-hydroxyphenyl)-5,9(11)-estradien * als weiße Schäume isoliert.
Exemplarisch ist die Darstellung einiger weiterer Verbindungen analog der obigen Vorschrift in der nachstehenden Tabelle aufgeführt :

| **Produkt** | **Ausbeute [%]** | **Physikalische Daten** |
|---|---|---|
| 3,3;17,17-Bis-(ethylendioxy)-11β-(4-methoxyphenyl)-5-estren | 81 | Fp. = 187-188 °C (Diisopropylether) [α]²⁰_{D} = +2° (CHCl₃;c=0,51) |
| 3,3;17,17-Bis-(ethylendioxy)-11β-(4-methylphenyl)-5-estren | 89 | Fp. = 200-201 °C (Diisopropylether) [α]²⁰_{D} = + 10 ° (CHCl₃;c=0,375) |
| 3,3;17,17-Bis(ethylendioxy)-11β-[4-(2-propenyl)-phenyl)-5-estren | 85 | ¹H,NMR (CDCl₃ δ [ppm]: 7,25 (2H,d J=9,5Hz,H-aromatisch);7,04 (2H,d J=9,5 Hz,H-aromatisch);5,9-6,07 (1H,m,H-CH=);5,53 (1H,d J=5Hz breit); 5,0-5,1(2H,m, H-CH₂=);3,42(1H,tr J=5,5 Hz breit,H-11);3,34(2H,d J=6Hz.H-CH₂-ar);0,56(3H, s,H-18) |
| * Fp. = 168-170 °C (Ethylacetat); [α]²⁰_{D} = - 11° (CHCl₃;c=0,505) | | |

d) 11β-(4-Methoxyphenyl)-4-estren-3,17-dion
19,2 g 3,3;17,17-Bis-(ethylendioxy)-11β-(4-methoxyphenyl)-5-estren werden in 500 ml Aceton gelöst und unter Schutzgas mit 12,5 ml 4 n wäßriger Salzsäure versetzt. Nach 2-stündigem Rühren bei 40°C wird das Reaktionsgemisch auf kalte gesättigte Natriumhydrogencarbonatlösung gegossen und die wäßrige Phase mehrmals mit Methylenchlorid extrahiert. Die vereinigten organischen Phasen werden über Natriumsulfat getrocknet und am Vakuum eingeengt. Der Rückstand wird an Kieselgel mit einem Gemisch aus Ethylacetat/Hexan chromatographiert. Man erhält 12,94 g 11β-(4-Methoxyphenyl)-4-estren-3,17-dion als weißen Schaum.
Fp. = 155-156 °C (Diisopropylether)
[α]²⁰_{D} = + 169 ° (CHCl₃;c=0.505)
e) 3-Ethoxy-11β-(4-methoxyphenyl)-3,5-estradien-17-on
10 g 11β-(4-Methoxyphenyl)-4-estren-3,17-dion werden in einem Gemisch aus 260 ml absolutem Methylenchlorid, 29 ml Ethanol und 26,4 ml Orthoameisensäuretriethylester vorgelegt und bei 0 °C mit 100 mg p-Toluolsulfonsäure (Monohydrat) versetzt. Anschließend wird bei Eisbadtemperatur 3,5 Stunden nachgerührt, dann das Reaktionsgemisch mit einem Überschuß an gesättigter Natriumhydrogencarbonatlösung versetzt und die wäßrige Phase mehrmals mit Methylenchlorid extrahiert. Die vereinigten organischen Phasen werden über Natriumsulfat getrocknet und am Vakuum eingeengt. Das Rohprodukt wird direkt in den folgenden Reaktionsschritt eingesetzt. Es werden so 10,8 g rohes 3-Ethoxy-11β-(4-methoxyphenyl)-3,5-estradien-17-on isoliert.
f) 3-Ethoxy-11β-(4-methoxyphenyl)-17α-(prop-1-inyl)-3,5-estradien-17-ol
1,2 l absolutes Tetrahydrofuran werden bei 0 °C mit Propin gesättigt. Anschließend werden zu dieser Lösung 165 ml einer 1,6 m n-Butyllithiumlösung (Hexan) langsam ohne größere Temperaturerhöhung zugetropft. Nach 15-minütigem Nachrühren tropft man bei Eisbadkühlung langsam eine Lösung des unter e) dargestellten Ketons (10,8 g), gelöst in 130 ml absolutem Tetrahydrofuran, zu dieser Reaktionsmischung und läßt sie 60 Minuten nachrühren. Danach wird das Reaktionsgemisch auf Wasser gegossen, die wäßrige Phase mit Ethylacetat extrahiert und die organische Phase mit Natriumchloridlösung gewaschen. Nach Trocknen über Natriumsulfat und Einengen der organischen Phase am Vakuum wird der Rückstand (11,8 g), wie in der nachfolgenden Vorschrift beschrieben, direkt weiter umgesetzt.
g) 6β-Brom-17β-hydroxy-11β-(4-methoxyphenyl)-17α-(prop-1-inyl)-4-estren-3-on
11,8 g des unter f) dargestellten Rohprodukts werden in einem Gemisch aus 120 ml 80%-iger wäßriger Dioxanlösung und 56 ml 10%-iger wäßriger Natriumacetatlösung suspendiert. Zu dieser Suspension werden 3,8 g 1,3-Dibrom-5,5-dimethylhydantoin portionsweise bei 0 °C zugegeben. Dabei geht das Steroid langsam in Lösung. Nach 330-minütiger Reaktionszeit wird das Reaktionsgemisch auf Wasser gegossen und die wäßrige Phase mit Methylenchlorid extrahiert. Die vereinigten organischen Phasen werden mit gesättigter Natriumthiosulfatlösung und Wasser gewaschen, über Natriumsulfat getrocknet und am Vakuum eingeengt. Man erhält 13,4 g rohes 6β-Brom-17β-hydroxy-11β-(4-methoxyphenyl)-17α-(prop-1-inyl)-4-estren-3-on, das direkt in den nächsten Reaktionsschritt eingesetzt wird.
h) 17β-Hydroxy-11β-(4-methoxyphenyl)-17α-(prop-1-inyl)-4,6-estradien-3-on
13,4 g der unter g) dargestellten Bromverbindung werden in 115 ml absolutem Dimethylformamid gelöst, unter Schutzgas mit 3,9 g Lithiumcarbonat und 5,75 g Lithiumbromid versetzt und eine Stunde bei 100 °C gerührt. Nach Abkühlen der Reaktionsmischung auf Raumtemperatur wird sie auf Wasser gegossen, die wäßrige Phase mit 4n Salzsäure neutralisiert und die wäßrige Phase mehrmals mit Ethylacetat extrahiert. Die vereinigten organischen Phasen werden mehrmals mit gesättigter Natriumchloridlösung gewaschen, über Natriumsulfat getrocknet und am Vakuum eingeengt. Der Rückstand wird an Kieselgel mit einem Gemisch aus Ethylacetat/Hexan chromatographiert. Es werden 5,6 g der Titelverbindung als gelblicher Schaum isoliert.
¹H-NMR (CDCl₃) δ [ppm]: 7,32 (2H,d J=9Hz,H-aromatisch); 6,83 (2H,d J=9Hz,H-aromatisch); 6,21-6,37 (2H,m,H-6 und H-7); 5,8 (1H,s,H-4); 3,8 (3H,s,H-Methylether); 3,4 (1H,tr breit J=6Hz,H-11); 1,88 (3H,s,H-Propin); 0,63 (3H,s,H-18).

### Beispiel 2

### 17β-Hydroxy-11β-(4-methoxyphenyl)-7β-methyl-17α-(prop-1-inyl)-4-estren-3-on und

### Beispiel 3

### 17β-Hydroxy-11β-(4-methoxyphenyl)-7α-methyl-17α-(prop-1-inyl)-4-estren-3-on

78 ml einer 1,6 m Lösung von Methyllithium in Diethylether werden in 120 ml Ether bei 0 °C vorgelegt, mit 11,8 g Kupfer-(I)-iodid versetzt und eine Stunde nachgerührt. Anschließend wird zum Dimethylcuprat eine Lösung von 2,5 g des unter Beispiel 1 h) dargestellten Dienons in 30 ml absolutem Tetrahydrofuran zugetropft. Nach einer Stunde Reaktionszeit wird der Ansatz in verdünnte wäßrige Ammoniaklösung gegossen und die wäßrige Phase mehrmals mit Ethylacetat extrahiert. Waschen der vereinigten organischen Phasen mit gesättigter Kochsalzlösung, Trocknen über Nätriumsulfat und Einengen am Vakuum führen zum Rohprodukt. Das Rohprodukt wird in 60 ml Aceton aufgenommen, mit 3 ml 4 n wäßriger Salzsäure versetzt und eine Stunde bei Raumtemperatur gerührt. Anschließend wird das Reaktionsgemisch auf gesättigte Natriumhydrogencarbonatlösung gegossen und die wäßrige Phase mehrmals mit Ethylacetat extrahiert. Die vereinigten organischen Phasen werden mit gesättigter Natriumchloridlösung gewaschen, über Natriumsulfat getrocknet und am Vakuum eingeengt. Der Rückstand wird an Kieselgel mit einem Gemisch aus Ethylacetat/Hexan chromatographiert. Es werden 1,35 g Gemisch der Titelverbindungen als weißer Schaum isoliert. Auftrennung über HPLC an Hypersil (ODS = Octadecylsilan, 5µ) mit einem Gemisch aus Wasser und Acetonitril führen zu 773 mg 17β-Hydroxy-11β(4-methoxyphenyl)-7α-methyl-17α-(prop-1-inyl)-4-estren-3-on und 235 mg 17β-Hydroxy-11β-(4-methoxyphenyl)-7β-methyl-17α-(prop-1-inyl)-4-estren-3-on als weiße Schäume.

Verbindung 2: ¹H-NMR (CDCl₃) δ [ppm]: 7,34 (2H,d J=9Hz,H-aromatisch); 6,83 (2H,d J=9Hz,H-aromatisch); 5,83 (1H,s,H-4); 3,8 (3H,s,H-Methylether; 3,4 (1H,tr breit J=5,5Hz,H-11); 1,89 (3H,s,H-Propin); 1,17 (3H,d J=6Hz,H-7-Methylgruppe); 0,59 (3H,s,H-18).

Verbindung 3: ¹H-NMR (CDCl₃) δ [ppm]: 7,32 (2H,d J=9Hz,H-aromatisch); 6,82 (2H,d j=9Hz,H-aromatisch); 5,87 (1H,s,H-4); 3,8 (3H,s,H-Methylether); 3,4 (1H,tr breit J=5,5Hz,H-11); 1,89 (3H,s,H-Propin); 0,87 (3H,d J=7,5Hz,H-7-Methylgruppe); 0,65 (3H,s,H-18).

### Beispiel 4

### 11β-(4-Acetylphenyl)-17β-hydroxy-17α-(3-hydroxyprop-1-Z)-enyl)-6β-methyl-4-estren-3-on

a) 3,3;17,17-Bis-(ethylendioxy)-11β-(4-methoxyphenyl)-5α,6α-epoxy-estran
   20 g des in Beispiel 1 c) dargestellten Diketals werden in 200 ml Methylenchlorid gelost. Man versetzt mit 0,1 ml Pyridin, kühlt auf 0°C und gibt 0,9 ml Hexachloraceton hinzu. Anschließend werden 9,2 ml einer 30%igen wäßrigen Wasserstoffperoxidlösung hinzugetropft. Es wird 7 Tage bei Raumtemperatur nachgerührt. Dann wird die Reaktionslösung vorsichtig bei leichter Kühlung mit gesättigter Natriumthiosulfatlösung versetzt. Man trennt die wäßrige Phase ab, extrahiert sie mehrmals mit Methylenchlorid und vereinigt die organischen Phasen. Sie werden mit gesättigter Natriumchloridlösung gewaschen, über Natriumsulfat getrocknet und am Vakuum eingeengt. Das Rohprodukt wird durch Chromatographie an Kieselgel mit einem Gemisch aus Ethylacetat und Hexan aufgereinigt. Man erhält 16,6 g 3,3;17,17-Bis-(ethylendioxy)-11β-(4-methoxyphenyl)-5α,6α-epoxy-estran als weißen Schaum.
   Fp. = 239-241 °C (Ethylacetat)
   [α]²⁰_{D} = - 44° (CHCl₃;c=0,505)
b) 3,3;17,17-Bis-(ethylendioxy)-11β-(4-methoxyphenyl)-6β-methyl-estran-5α-ol
   15 g des unter a) dargestellten Epoxids werden unter Schutzgas in 200 ml absolutem Tetrahydrofuran gelöst, mit 138 ml einer 3 m Methylmagnesiumchloridlösung (Tetrahydrofuran) versetzt und anschließend 22 Stunden bei Raumtemperatur gerührt. Danach wird das Reaktionsgemisch auf gesättigte Ammoniumchloridlösung gegossen und die wäßrige Phase mit Ethylacetat extrahiert. Die vereinigten Organischen Phasen werden mit gesättigter Natriumchloridlösung gewaschen, über Matriumsulfat getrocknet und am Vakuum eingeengt. Das Rohprodukt wird durch Chromatographie an Kieselgel mit einem Gemisch aus Ethylacetat und Hexan aufgereinigt. Man erhält 13,8 g 3,3;17,17-Bis-(ethylen-dioxy)-11β-(4-methoxyphenyl)-6β-methyl-estran-5α-ol als weißen Schaum.
   Fp. = 168-169 °C (Ethylacetat)
   [α]²⁰_{D} = - 36° (CHCl₃;c=0,505)
c) 3,3;17,17-Bis-(ethylendioxy)-11β-(4-hydroxyphenyl)-6β-methyl-estran-3α-ol
   13 g des unter b) dargestellen Anisols werden in 130 ml absolutem Dimethylformamid gelöst, mit 7,3 g Natriummethanthiolat versetzt und das Reaktionsgemisch 1,5 Stunden zum Rückfluß erhitzt. Nach Abkühlen wird es auf Wasser gegossen und die wäßrige Phase mit Ethylacetat extrahiert. Die vereinigten organischen Phasen wäscht man mehrmals mit gesättigter Natriumchloridlösung, trocknet sie über Natriumsulfat und engt am Vakuum ein. Der Rückstand wird an Kieselgel mit einem Gemisch aus Ethylacetat/Hexan chromatographiert und man erhält 8,95 g 3,3;17,17-Bis-(ethylendioxy)-11β-(4-hydroxyphenyl)-6β-methyl-estran-5α-ol als weißen Schaum.
d) 3,3;17,17-Bis-(ethylendioxy)-6β-methyl-11β-(4-trifluormethylsulfonyloxyphenyl)estran-5α-ol
   8,5 g des unter c) dargestellten Phenols werden unter Schutzgas zusammen mit 11,75 g 4-Dimethylaminopyridin in 175 ml absolutem Methylenchlorid gelöst, auf -78 °C gekühlt und mit 3,85 ml Trifluormethansulfonsäureanhydrid, gelöst in 23 ml absolutem Methylenchlorid, versetzt, Nach 1,5-stündigem Nachrühren wird das Reaktionsgemisch auf gesättigte Natriumhydrogencarbonatlösung gegossen und die wäßrige Phase mehrmals mit Methylenchlorid extrahiert. Die vereinigten organischen Phasen wäscht man mit gesättigter Natriumchloridlösung, trocknet sie über Natriumsulfat und engt am Vakuum ein. Der Rückstand wird an Kieselgel mit einem Gemisch aus Ethylacetat/Hexan chromatographiert und man erhält 7,76 g 3,3;17,17-Bis-(ethylendioxy)-6β-methyl-11β-(4-trifluormethylsulfonyloxyphenyl)-estran-5α-ol als weißen Schaum.
   [α]²⁰_{D} = - 28 ° (CHCl₃;c=0,505)
c) 3,3-(Ethylendioxy)-5α-hydroxy-6β-methyl-11β-(4-trifluormethylsulfonyloxyphenyl)estran-17-on
   19 g Kieselgel werden in 38 ml Methylenchlorid suspendiert mit 194 ml gesättigter Oxalsäurelösung versetzt und 15 Minuten nachgerührt. Zu dieser Suspension werden 7,7 g des unter d) dargestellten Triflats gegeben und das Reaktionsgemisch bei Raumtemperatur 2,5 Stunden nachgerührt. Anschließend wird es über eine Fritte abgesaugt, der Frittenrückstand mit Methanol/Methylenchlorid nachgewaschen und das so erhaltene Filtrat mit gesättigter Natriumhydrogencarbonatlösung ausgeschüttelt. Die organische Phase wird über Natriumsulfat getrocknet und am Vakuum eingeengt. Der Rückstand wird an Kieselgel mit einem Gemisch aus Ethylacetat/Hexan chromatographiert. Man erhält 4,08 g 3,3-(Ethylendioxy)-5α-hydroxy-6β-methyl-11β-(4-trifluormethylsulfonyloxyphenyl)-estran-17-on als weißen Schaum.
   [α]²⁰_{D} = 42° (CHCl₃;c=0,53)
f) 11β-[4-(1-Ethoxyvinyl)-phenyl]-3,3-(ethylendioxy)-5α-hydroxy-6β-methyl-estran-17-on
   4 g des unter e) dargeslellten Triflats werden in 35 ml absolutem Dioxan gelöst und mit 585 mg Lithiumchlorid und 0,8 g Tetrakistriphenylphosphinpalladium versetzt. Nach fünfminütigem Nachrühren wird das Reaktionsgemisch mit 2,91 ml Tri-n-butyl-(1-ethoxyvinyl)-zinn versetzt, 1 Stunde bei Rückfluß unter Schutzgas gerührt, auf Raumtemperatur abgekühlt und mit Ethylacetat verdünnt. Nach Filtration über Celite und Waschen des Filterrückstandes mit Ethylacetat wird die organische Phase mit gesättigter Natriumchloridlösung gewaschen, über Natriumsulfat getrocknet und am Vakuum eingeengt. Das Rohprodukt wird direkt in den nächsten Reaktionnchritt eingesetzt.
g) 11β-[4-(1-Ethoxyvinyl)phenyl]-3,3-(ethylendioxy)-17α-(3-hydroxyprop-1-inyl)-6β-methyl-estran-5α,17β-diol
   Das unter f) erhaltene Rohprodukt wird in 100 ml absolutem Tetrahydrofuran gelöst und nacheinander bei 0 °C mit 16 g Kaliumethylat und 5,6 ml Propargylalkohol versetzt. Nach einstündigem Nachrühren bei 0 °C und dreistündigem bei Raumtemperatur wird das Reaktionsgemisch auf gesättigte Ammoniumchloridlösung gegossen und die wäßrige Phase mit Ethylacetat extrahiert. Die vereinigten organischen Phasen werden mit gesättigter Natriumchloridlösung gewaschen, über Natriumsulfat getrocknet und am Vakuum eingeengt. Das Rohprodukt wird durch Chromatographie an Kieselgel mit einem Gemisch aus Ethylacetat und Hexan aufgereinigt. Es werden 1,96 g 11β-[4-(1-Ethoxyvinyl)-phenyl]-3,3-(ethylendioxy)-17α-(3-hydroxyprop-1-inyl)-6β-methyl-estran-5α,17β-diol als weißer Schaum erhalten.
h) 11β-(4-Acetylpheny)-5α,17β-dihydroxy-17α-(3-hydroxyprop-1-inyl)-6β-methyl-estran-3-on
   1,9 g des unter g) dargestellen Diols werden in 95 ml Aceton gelöst und unter Schutzgas mit 9,5 ml 4 n wäßriger Salzsäure versetzt. Nach 2-stündigem Rühren bei 0°C wird das Reaktionsgemisch auf kalte gesättigte Natriumhydrogencarbonatlösung gegossen und die wäßrige Phase mehrmals mit Methylenchlorid extrahiert. Die vereinigten organischen Phasen werden über Natriumsulfat getrocknet und am Vakuum eingeengt Der Rückstand wird an Kieselgel mit einem Gemisch aus Ethylacetat/Hexan chromatographiert. Man erhält 1,42 g 11β-(4-Acetylphenyl)-5α,17β-dihydroxy-17α-(3-hydroxyprop-1-inyl)-6β-methyl-estran-3-on als weißen Schaum.
i) 11β-(4-Acetylphenyl)-5α,17β-dihydroxy-17α-(3-hydroxyprop-1(Z)-enyl-6β-methyl-estran-3-on
   1,3 g des unter h) dargestellten Acetylens werden in 13 ml Tetrahydrofuran gelöst, mit 1,3 ml Pyridin versetzt und unter Verwendung von 130 mg Palladium (10%-ig) auf Bariumsulfat als Katalysator bei Normaldruck hydriert. Nach Aufnahme eines Äquivalents Wasserstoff wird das Reaktionsgemisch über Celite filtiert, der Filterückstand mit Ethylacetat nachgewaschen und das Filtrat am Vakuum eingeengt. Der Rückstand wird an Kieselgel mit einem Gemisch aus Hexan/Ethylacetat chromatographiert, und man erhält 1,12 g 11β-(4-Acetylphenyl)-5α,17β-dihydroxy-17α-(3-hydxoxyprop-1(Z)-enyl)-6β-methylestran-3-on als weißen Schaum.
j) 11β-(4-Acetylphenyl)-17β-hydroxy-17α-(3-hydroxyprop-1(Z)-enyl)-6β-methyl-4-estren-3-on
   1,1 g des unter i) dargestellten Ketons werden unter Schutzgas in 45 ml Ethanol gelöst und mit 2,2 ml 0,1 n wäßriger Natronlauge versetzt. Nach 6-stündigem Rühren wird das Reaktionsgemisch mit Wasser versetzt, mit 4 n wäßriger Salzsäure neutralisiert und die wäßrige Phase mehrmals mit Essigester extrahiert. Die vereinigten organischen Phasen werden mit gesättigter Natriumchloridlösung gewaschen, über Natriumsulfat getrocknet und am Vakuum eingeengt. Der Rückstand wird an Kieselgel mit einem Gemisch aus Ethylacetat/Hexan chromatographiert. Man erhält 875 mg der Titelverbindung als weißen Schaum.
   ¹H-NMR (CDCl₃) δ [ppm]: 7,91 (2H,d J=9Hz,H-aromatisch); 7,3 (2H,d J=9Hz,H-aromatisch); 5,9 (1H,d J=1Hz,H-4); 5,59-5,8 (2H,m,H-20 und H-21); 4,26 (2H,d J=5,5Hz,H-22); 3,42 (1H,tr breit J=5,5Hz,H-11); 2,6 (3H,s,H-Acylgruppe); 1,36 (3H,d J=7Hz,H-6-Methylgruppe); 0,68 (3H,s,H-18).

### Beispiel 5

### 17β-Hydroxy-11β-(4-acetylphenyl)-17α-(3-hydroxyprop-1(Z)-enyl)-6α-methyl-4-estren-3-on

600 mg der unter Beispiel 4j) dargestellten 11β-(4-Acetylphenyl)-17β-hydroxy-17α-(3-hydroxyprop-1(Z)-enyl)-6β-methyl-4-estren-3-on werden analog der Vorschrift Beispiel 1 d) mit 4n wäßriger Salzsäure in Aceton behandelt. Nach Aufarbeinung wird das Rohprodukt über HPLC an Hypersil (ODS 5µ) mit einem Gemisch aus Wasser und Methanol gereinigt. Es werden 385 mg der Titelverbindung als weißer Schaum isoliert.
¹H-NMR (CDCl₃) δ [ppm]: 7,87 (2H,d J=9Hz,H-aromatisch); 7,55 (2H,d J=9Hz,H-aromatisch); 5,87 (1H,s,H-4); 5,55-5,75 (2H,m,H-20 und H-21); 4,26 (2H,d J=5,5Hz,H-22); 3,42 (1H,dd breit J₁=5,5 und J₂=11Hz,H-11); 2,58 (3H,s,H-Acylgruppe); 1,15 (3H,d J=5,5Hz, H-6-Methylgruppe); 0,65 (3H,s,H-18).

### Beispiel 6

### 17β-Hydroxy-17α-(3-hydroxyprop-1-inyl)-6β-methyl-11β-[4-(3-pyridinyl)-phenyl]-4-estren-3-on

a) 3,3-(Ethylendioxy)-5α-hydroxy-6β-methyl-11β-[4-(3-pyridinyl)-phenyl]-estran-17-on 10 g des nach der Vorschrift Beispiel 4 e) dargestellten 3,3-(Ethylendioxy)-5α-hydroxy-6β-methyl-11β-(4-trifluormethylsulfonylxyphenyl)-estran-17-on werden in einem Gemisch aus 80 ml Toluol und 35 ml Ethanol gelöst und nacheinander mit 1 g Palladiumtetrakistriphenylphosphin, 1,47 g Lithiumchlorid, 22,5 ml 2 m Natriumcarbonatlösung und 2,8 g Diethyl-(3-pyridinyl)-boran versetzt. Das Reaktionsgemisch wird dann 3 Stunden bei 110 °C gerührt, auf Raumtemperatur abgekühlt und mit gesättigter Natriumchloridlösung versetzt. Die organische Phase wird abgetrennt, nacheinander mit 5 %-iger Natronlauge und Wasser gewaschen, über Natriumsulfat getrocknet und am Vakuum eingeengt. Der Rückstand wird an Kieselgel mit einem Gemisch aus Ethylacetat/Hexan chromatographiert. Man erhält 7,53 g 3,3-(Ethylendioxy)-5α-hydroxy-6β-methyl-11β-[4-(3-pyridyl)-phenyl]-estran-17-on als weißen Schaum.
   [α]²⁰_{D} = 58 °(CHCl₃;c=0,52)
b) 3,3-(Ethylendioxy)-17α-(3-hydroxyprop-1-inyl)-6β-methyl-11β-[4-(3-pyridinyl)-phenyl]-estran-5α,17β-diol
   7 g des unter a) dargestellten Ketons werden analog der unter Beispiel 4 g) beschriebenen Vorschrift mit Kaliumethylat und Propargylalkahol zu 5,83 g 3,3-(Ethylendioxy)-17α-(3-hydroxyprop-1-inyl)-6β-methyl-11β-[4-(3-pyridinyl)-phenyl]-estran-5α,17β-diol umgesetzt. Es wird als weißer Schaum isoliert.
c) 5α,17β-Dihydroxy-17α-(3-hydroxyprop-1-inyl)-6β-methyl-11β-[4-(3-pyridinyl)-phenyl]-estran-3-on
   5 g des unter b) dargestellten Triols werden analog der unter Beispiel 4 h) beschriebenen Vorschrift mit 4 n wäßriger Salzsäure in Aceton bei 0 °C zu 4,02 g 5α,17β-Dihydroxy-17α-(3-hydroxyprop-1-inyl)-6β-methyl-11β-[4-(3-pyridinyl)-phenyl]-estran-3-on umgesetzt. Es wird als weißer Schaum isoliert.
   [α]²⁰_{D} = - 62 °(CHCl₃;c=0,505)
d) 17β-Hydroxy-17α-(3-hydroxyprop-1-inyl)-6β-methyl-11β-[4-(3-pyridiny)-phenyl]-4-estren-3-on
   1,75 g des unter c) dargestellten Ketons werden analog der unter Beispiel 4 j) beschriebenen Vorschrift mit 2 n wäßriger Natronlauge in Ethanol umgesetzt. Es werden 1,45 g der Titelverbindung als weißer Schaum isoliert.
   ¹H-NMR (CDCl₃) δ [ppm]: 8,87 (1H,s breit,H-heteroaromatisch); 8,59 (1H,d J=5Hz, H-heteroaromatisch); 7,9 (1H,dtr, J₁=1,5 und J₂=9Hz,H-heteroaromatisch); 7,48-7,63 (4H,m,H-aromatisch); 7,38 (1H,dd J₁=5 und J₂=9Hz,H-heteroaromatisch); 5,91 (1H,d J=1Hz,H-4); 5,36 (2H,s,H-22); 3,46 (1H,tr breit J=5,5Hz,H-11); 1,38 (3H,d J=6,5Hz, H-6-Methylgruppe); 0,7 (3H,s,H-18).

### Beispiel 7

### 17β-Hydroxy-17α-(3-hydroxyprop-1(Z)-enyl)-6β-methyl-11β-[4-(3-pyridinyl)-phenyl]-4-estren-3-on

a) 5α,17β-Dihydroxy-17α-(3-hydroxyprop-1(Z)-enyl)-6β-methyl-11β-[4-(3-pyridinyl)-phenyl]-estran-3-on
   2 g des unter 6 c) dargestellten Ketons werden analog der unter Beispiel 4 i) beschriebenen Vorschrift hydriert. Es werden 1,68 g 5α,17β-Dihydroxy-17α-(3-hydroxyprop-1(Z)-enyl)-6β-methyl-11β-[4-(3-pyridinyl)-phenyl]-estran-3-on als weißer Schaum isoliert.
b) 17β-Hydroxy-17α-(3-hydroxyprop-1(Z)-enyl)-6β-methyl-11β-[4-(3-pyridinyl)-phenyl]-4-estren-3-on
   1,5 g des unter a) dargestellten Ketons werden analog der unter Beispiel 4 j) beschriebenen Vorschrift mit 2 n wäßriger Natronlauge in Ethanol umgesetzt. Es werden 1,15 g der Titelverbindung als weißer Schaum isoliert.
   [α]²⁰_{D} = 62 ° (CHCl₃;c=0,5)
   ¹H-NMR (CDCl₃) δ [ppm]: 8,88 (1H,s breit,H-heteroaromatisch); 8,59 (1H,d J=5Hz, H-heteroaromatisch); 7,9 (1H,dtr J₁=1,5 und J₂=9Hz,H-heteroaromatisch); 7,49-7,57 (4H,m,H-aromatisch); 7,39 (1H,dd J₁=5 und J₂=9Hz,H-heteroaromatisch); 5,9 (1H,d J=1Hz,H-4); 5,6-5,8 (2H,m,H-20 und H-21); 5,27 (2H,d J=6Hz,H-22); 3,42 (1H,tr breit J=5,5Hz,H-11); 1,35 (3H,d J=6,5Hz,H-6-Methylgruppe); 0,75 (3H,s,H-18).

### Beispiel 8

### 17β-Hydroxy-17α-(3-hydroxyprop-1(Z)-enyl)-6α-methyl-11β-[4-(3-pyridinyl)-phenyl]-4-estren-3-on

800 mg des unter Beispiel 7 b) dargestellten 17β-Hydroxy-17α-(3-hydroxyprop-1(Z)-enyl)-6β-methyl-11β-[4-(3-pyridinyl)-phenyl]-4-estren-3-on werden analog der Vorschrift Beispiel 1 d) mit 4n wäßriger Salzsäure in Aceton behandelt. Nach Aufarbeitung wird das Rohprodukt über HPLC an Hypersil (ODS 5µ) mit einem Gemisch aus Wasser und Acetonitril gereinigt. Es werden 463 mg der Titelverbindung als weißer Schaum isoliert.
[α]²⁰_{D} = 80 ° (CHCl₃;c=0,505)
¹H-NMR (CDCl₃) δ [ppm]: 8,85 (1H,s breit,H-heteroaromatisch); 8,57 (1H,d J=5Hz, H-heteroaromatisch); 7,9 (1H,dtr J₁=1,5 und J₂=9Hz,H-heteroaromatisch); 7,45-7,57 (4H,m,H-aromatisch); 7,38 (1H,dd J₁=5 und J₂=9Hz,H-heteroaromatisch); 5,88 (1H,s, H-4); 5,6-5,8 (2H,m,H-20 und H-21); 5,28 (2H,d J=5Hz,H-22); 3,42 (1H,tr breit J=5,5Hz, H-11); 1,16 (3H,d J=5,5Hz,H-6-Methylgruppe); 0,73 (3H,s,H-18).

### Beispiel 9

### 11β-(4-Dimethylaminophenyl)-17β-hydroxy-17α-(prop-1-inyl)-4,6-estradien-3-on

a) 11β-(4-Dimethylaminophenyl)-4-estren-3,17-dion
   Analog der unter Beispiel 1 d) beschriebenen Vorschrift werden aus 10 g 3,3;17,17-Bis(ethylendioxy)-11β-(4-dimethylaminophenyl)-5-estren (Herstellung siehe EP-A 50343) 6,6 g der Titelverbindung als Rohprodukt erhalten.
   ¹H-NMR (CDCl₃) δ [ppm]: 7,27 (2H,d J=9Hz,H-aromatisch); 6,67 (2H,d J=9Hz,H-aromatisch); 5,88 (1H,s,H-4); 3,32 (1H,tr breit J=5,5Hz,H-11); 2,94 (6H,s,H-NMe₂); 0,70 (3H,s, H-18).
b) 11β-(4-Dimethylaminophenyl)-3-ethoxy-3,5-estradien-17-on
   Analog der unter Beispiel 1 e) beschriebenen Vorschrift werden aus 3,75 g 11β-(4-Dimethylaminophenyl)-4-estren-3,17-dion 4,04 g der Titelverbindung als Rohprodukt erhalten.
c) 11β(4-Dimethylaminophenyl)-3-ethoxy-17α-(prop-1-inyl)-3,5-estradien-17β-ol
   Analog der unter Beispiel 1 f) beschriebenen Vorschrift werden aus 4,04 g 11β-(4-Dimethylaminophenyl)-3-ethoxy-3,5-estradien-17-on 1,98 g der Titelverbindung als weißer Schaum erhalten.
d) 6β-Brom-11β-(4-dimethylaminophenyl)-17β-hydroxy-17α-(prop-1-inyl)-4-estren-3-on und 6β-Brom-11β-(3-Brom-4-dimethylaminophenyl)-17β-hydroxy-17α-(prop-1-inyl)-4-estren-3-on
   Analog der unter Beispiel 1 g) beschriebenen Vorschrift werden aus 1,9 g 11β-(4-Dimethylaminophenyl)-3-ethoxy-17α-(prop-1-inyl)-3,5-estradien-17β-ol mit 0,613 Äquivalenten 1,3-Dibrom-5,5-dimethylhydantoin 1,09 g und 635 mg der Titelverbindungen als gelbliche Schäume erhalten.
e) 11β-(4-Dimethylaminophenyl)-17β-hydroxy-17α-(prop-1-inyl)-4,6-estradien-3-on
   Analog der unter Beispiel 1 h) beschriebenen Vorschrift werden aus 0,9 g 6β-Brom-11β-(4-dimethylaminophenyl)-17β-hydroxy-17α-(prop-1-inyl)-4-estren-3-on 465 mg der Titelverbindung als gelblicher Schaum erhalten.
   ¹H-NMR (CDCl₃) δ [ppm]: 7,25 (2H,d J=9Hz,H-aromatisch); 6,67 (2H,d J=9Hz,H-aromatisch); 6,2-6,33 (2H,m,H-6 und H-7); 5,8 (1H,s,H-4); 3,36 (1H,tr breit J=6Hz,H-11); 2,95 (6H,s,H-Methylgruppen am Stickstoff); 1,88 (3H,s,H-Propin); 0,67 (3H,s,H-18).
   [α]²⁰_{D} = -23° (CHCl₃;c=0,505)

### Beispiel 10

### 11β-(3-Brom-4-dimethylaminophenyl)-17β-hydroxy-17α-(prop-1-inyl)-4,6-estradien-3-on

Analog der unter Beispiel 1 h) beschriebenen Vorschrift werden aus 0,55 g 6β-Brom-11β-(3-Brom-4-dimethylaminophenyl)-17β-hydroxy-17α-(prop-1-inyl)-4-estren-3-on 235 mg der Titelverbindung als gelblicher Schaum erhalten.
¹H-NMR (CDCl₃) δ [ppm]: 7,53 (1H,d J=1Hz,H-aromatisch); 7,31 (1H,dd J=1Hz und J₁=9Hz,H-aromatisch); 6,98 (1H,d J₁=9Hz,H-aromatisch); 6,21-6,34 (2H,m,H-6 und H-7); 5,81 (1H,s,H-4); 3,36 (1H,tr breit J=6Hz,H-11); 2,8 (6H,s,H-Methylgruppen am Stickstoff); 1,88 (3H,s,H-Propin); 0,63 (3H,s,H-18).
[α]²⁰_{D} = -30° (CHCl₃;c=0,515

### Beispiel 11

### 11β-(4-Acetylphenyl)-17β-hydroxy-17α-(prop-1-inyl)-4,6-estradien-3-on

a) 3,3,17,17-Bis-(ethylendioxy)-11β-(4-hydroxyphenyl)-5-estren 130 g der in Beispiel 1 c) hergestellten Verbindung werden in 1,4 l absolutem Dimethylformamid gelöst, mit 78,1 g Natriummethanthiolat versetzt und das Reaktionsgemisch 3 Stunden zum Rückfluß erhitzt. Nach Abkühlen wird es auf Wasser gegossen und die wässrige Phase mit Ethylacetat extrahiert. Die vereinigten organischen Phasen wäscht man mehrmals mit gesättigter Natriumchloridlösung, trocknet sie über Natriumsulfat und engt am Vakuum ein. Der Rückstand wird an Kieselgel mit einem Gemisch aus Ethylacetat/Hexan chromatographiert und man erhält 76,9 g 3,3;17,17-Bis-(ethylendioxy)-11β-(4-hydroxyphenyl)-5-estren als weißen Schaum.
   Fp. = 224-225 °C (Diisopropylether)
   [α]²⁰_{D} = + 1.5 ° (CHCl₃;c=0.505)
b) 3,3;17,17-Bis-(ethylendioxy)-11β-(4-trifluormethylsulfonyloxyphenyl)-5-estren
   Analog der unter Beispiel 4 d) beschriebenen Vorschrift werden aus 76,1 g 3,3;17,17-Bis-(ethylendioxy)-11β-(4-hydroxyphenyl)-5-estren 73,5 g der Titelverbindung als weißer Schaum erhalten.
c) 11β-(4-Trifluormethylsulfonyloxyphenyl)-4-estren-3,17-dion
   Analog der unter Beispiel 1 d) beschriebenen Vorschrift werden aus 73 g 3,3;17,17-Bis-(ethylendioxy)-11β-(4-trifluormethylsulfonyloxyphenyl)-5-estren 51,3 g der Titelverbindung als Kristallisat erhalten.
d) 3-Ethoxy-11β-(4-trifluormethylsulfonyloxyphenyl)-3,5-estradien-17-on
   Analog der unter Beispiel 1 e) beschriebenen Vorschrift werden aus 50 g 11β-(4-Trifluormethylsulfonyloxyphenyl)-4-estren-3,17-dion 53,5 g der Titelverbindung als Rohprodukt erhalten.
e) 3-Ethoxy-17α-(prop-1-inyl)-11β-(4-trifluormethylsulfonyloxyphenyl)-3,5-estradien-17β-ol
   Analog der unter Beispiel 1 f) beschriebenen Vorschrift werden aus 31,7 g 3-Ethoxy-11β-(4-trifluormethylsulfonyloxyphenyl)-3,5-estradien-17-on 34,1 g der Titelverbindung als Rohprodukt erhalten.
f) 6β-Brom-17β-hydroxy-17α-(prop-1-inyl)-11β-(4-trifluormethylsulfonyloxyphenyl)-4-estren-3-on
   Analog der unter Beispiel 1 g) besbriebenen Vorschrift werden aus 34,1 g 3-Ethoxy-17α-(prop-1-inyl)-11β-(4-trifluormethylsulfonyloxyphenyl)-3,5-estradien-17β-ol 17,2 g der Titelverbindung als weißer Schaum erhalten.
   Fp. = 166 °C (Diisopropylether)
   [α]²⁰_{D} = - 73 ° (CHCl₃;c=0.505)
g) 17β-Hydroxy-17α-(prop-1-inyl)-11β-(4-trifluormethylsulfonyloxyphenyl)-4,6-estradien-3-on
   Analog der unter Beispiel 1 h) beschriebenen Vorschrift werden aus 11,3 g 6β-Brom-17β-Hydroxy-17α-(prop-1-inyl)-11β-(4-trifluormethylsulfonyloxyphenyl)-4-estren-3-on 9,1 g der Titelverbindung als gelblicher Schaum erhalten.
   Fp. = 212 °C (Diisopropylether)
   [α]²⁰_{D} = 46° (CHCl₃;c=0.5)
h) 11β-(4-Acetylphenyl)-17β-hydroxy-17α-(prop-1-inyl)-4,6-estradien-3-on
   Analog der unter Beispiel 4 f) und 4 h) beschriebenen Vorschriften werden ans 1 g 17α-(prop-1-inyl)-11β-(4-trifluormethylsulfonyloxyphenyl)-4,6-estradien-17β-ol 654 mg der Titelverbindung als gelblicher Schaum erhalten.
   ¹H-NMR (CDCl₃) δ [ppm]: 7,9 (2H,d J=9Hz,H-aromatisch); 7,53 (2H,d J=9Hz,H-aromatisch); 6,23-6,35 (2H,m,H-6 und H-7); 5,83 (1H,s,H-4); 3,5 (1H,tr breit J=6Hz,H-11); 2,62 (3H,s,H-Methylketon); 1,88 (3H,s,H-Propin); 0,58 (3H,s,H-18).
   Fp. = 250 °C (Zersetzung) (Diisopropylether)
   [α]²⁰_{D} = -5,4 ° (CHCl₃;c=0,51)

### Beispiel 12

### 11β-[4-(3-Furanyl)-phenyl]-17β-hydroxy-17α-(prop-1-inyl)-4,6-estradien-3-on

Analog der unter Beispiel 4 f) beschriebenen Vorschrift werden aus 535 mg der in Beispiel 11 g) hergestellten Verbindung durch Kupplung mit 0,5 ml 3-Tri-n-butylstannylfuran 379 mg der Titelverbindung als gelblicher Schaum erhalten.
¹H-NMR (CDCl₃) δ [ppm]: 7,74 (1H,s breit,H-heteroaromatisch); 7,49 (1H,tr J=0,5Hz,H-heteroaromatisch); 7,42 (4H,s,H-aromatisch); 6,7 (1H,d J=0,5Hz,H-heteroaromatisch); 6,24-6,34 (2H,m,H-6 und H-7); 5,81 (1H,s,H-4); 3,45 (1H,tr breit J=6Hz,H-11); 1,89 (3H,s,H-Propin); 0,65 (3H,s,H-18).
Fp. = 200 °C (Diisopropylether)
[α]²⁰_{D} = 7° (CHCl₃;c=0,51)

### Beispiel 13

### 11β-[4-(4-Cyanphenyl)-phenyl]-17β-hydroxy-17α-(prop-1-inyl)-4,6-estradien-3-on

Analog der unter Beispiel 6 a) beschriebenen Vorschrift werden aus 535 mg der in Beispiel 11 g) hergestellten Verbindung durch Kupplung mit 4-Cyanphenylboronsäure 471 mg der Titelverbindung als gelblicher Schaum erhalten.
¹H-NMR (CDCl₃) δ [ppm]: 7,67-7,76 (4H,m,H-aromatisch); 7,52 (4H,s,H-aromatisch); 6,24-6,37 (2H,m,H-6 und H-7); 5,82 (1H,s,H-4); 3,5 (1H,tr breit J=6Hz,H-11); 1,88 (3H,s,H-Propin); 0,65 (3H,s,H-18).
Fp. = 276 °C (Zersetzung) (Diisopopylether)
[α]²⁰_{D} = 73 ° (CHCl₃;c=0,51)

### Beispiel 14

### 17β-Hydroxy-11β-[4-(4-methylthiophenyl)-phenyl]-17α-(prop-1-inyl)-4,6-estradien-3-on

Analog der unter Beispiel 6 a) beschriebenen Vorschrift werden aus 2 g der in Beispiel 11 g) hergestellten Verbindung durch Kupplung mit 4-Methylthiophenylboronsäure 1,7 g der Titelverbindung als gelblicher Schaum erhalten.
¹H-NMR (CDCl₃) δ [ppm]: 7,55 (2H,d J=9Hz,H-aromatisch); 7,43-7,55 (4H,m,H-aromatisch); 7,32 (2H,d J=9Hz,H-aromatisch); 6,25-6,35 (2H,m,H-6 und H-7); 5,82 (1H,s,H-4); 3,48 (1H,tr breit J=6Hz,H-11); 2,53 (3H,s,H-Methylthioether); 1,88 (3H,s,H-Propin); 0,65 (3H,s,H-18).

### Beispiel 15

### 11β-[4-(4-Acetylphenyl)-phenyl]-17β-hydroxy-17α-(prop-1-inyl)-4,6-estradien-3-on

a) 17β-Hydroxy-17α-(prop-1-inyl)-11β-(4-tri-n-butylphenyl)-4,6-estradien-3-on
   12 g der in Beispiel 11 g) hergestellten Verbindung werden in 112 ml absolutem Dioxan gelöst und mit 1,42 g Lithiumchlorid und 0,7 g Tetrakistriphenylphosphinpalladium versetzt. Nach fünfminütigem Nachrühren wird das Reaktionsgemisch mit 16,8 ml Hexabutyldizinn versetzt, 2,5 Stunden bei Rückfluß unter Schutzgas gerührt, auf Raumtemperatur abgekühlt und mit Ethylacetat verdünnt. Nach Filtration über Celite und Waschen des Filterrückstandes mit Ethylacetat wird die organische Phase mit gesättigter Natriumchloridlösung gewaschen, über Natriumsulfat getrocknet und am Vakuum eingeengt. Chromatographie des Rückstandes an Kieselgel mit einem Gemisch aus Ethylacetat/Hexan ergeben 9,1 g der Titelverbindung als weißen Schaum.
   ¹H-NMR (CDCl₃) δ [ppm]: 7,3-7,37 (4H,m,H-aromatisch); 6,22-6,33 (2H,m,H-6 und H-7); 5,81 (1H,s,H-4); 3,42 (1H,tr breit J=6Hz,H-11); 1,89 (3H,s,H-Propin); 0,88 (9H,tr J=7,5Hz,H-terminale Methylgruppen); 0,63 (3H,s,H-18).
   Fp. = 154 °C
   [α]²⁰_{D} = 8° (CHCl₃;c=0,515)
b) 11β-[4-(4-Acetylphenyl)-phenyl]-17β-hydroxy-17α-(prop-1-inyl)-4,6-estradien-3-on
   Analog der unter Beispiel 4 f) beschriebenen Vorschrift werden aus 675 mg 17β-Hydroxy-17α-(prop-1-inyl)-11β-(4-tri-n-butylphenyl)-4,6-estradien-3-on 310 mg der Titelverbindung als gelblicher Schaum erhalten.
   ¹H-NMR (CDCl₃) δ [ppm]: 8,05 (2H,d J=9Hz,H-aromatisch); 7,7 (2H,d J=9Hz,H-aromatisch); 7,48-7,61 (4H,m,H-aromatisch); 6,25-6,35 (2H,m,H-6 und H-7); 5,82 (1H,s,H-4); 3,49 (1H,tr breit J=6Hz,H-11); 2,65 (3H,s,H-Methylketon); 1,88 (3H,s,H-Propin); 0,66 (3H,s,H-18).

### Beispiel 16

### 17β-Hydroxy-17α-(prop-1-inyl)-11β-[4-(5-pyrimidinyl)-phenyl]-4,6-estradien-3-on

Analog der unter Beispiel 4 f) beschriebenen Vorschrift werden aus 675 mg 17β-Hydroxy17α-(prop-1-inyl)-11β-(4-tri-n-butylphenyl)-4,6-estradien-3-on 285 mg der Titelverbindung als gelblicher Schaum erhalten.
¹H-NMR (CDCl₃) δ [ppm]: 9,2 (1H,s,H-heteroaromatisch); 8,98 (2H,s,H-heteroaromatisch); 7,5-7,62 (4H,m,aromatisch); 6,25-6,35 (2H,m,H-6 und H-7); 5,82 (1H,s,H-4); 3,51 (1H,tr breit J=6Hz,H-11); 1,89 (3H,s,H-Propin); 0,65 (3H,s,H-18).

### Beispiel 17

### 11β-[4-(3-Acetylphenyl)-phenyl]-17β-hydroxy-17α-(prop-1-inyl)-4,6-estradien-3-on

Analog der unter Beispiel 4 f) beschriebenen Vorschrift werden aus 675 mg 17β-Hydroxy-17α-(prop-1-inyl)-11β-(4-tri-n-butylphenyl)-4,6-estradien-3-on 301 mg der Titelverbindung als gelblicher Schaum erhalten.
¹H-NMR (CDCl₃) δ [ppm]: 8,19 (1H,tr J=0,5Hz,H-aromatisch); 7,93 (1H,dtr J=0,5Hz und J₁=8Hz,H-aromatisch); 7,8 (1H,dtr J=0,5Hz und J₁=8Hz,H-aromatisch); 7,46-7,59 (2H,m, H-aromatisch); 6,24-6,36 (2H,m,H-6 und H-7); 5,82 (1H,s,H-4); 3,5 (1H,tr breit J=6Hz, H-11); 2,67 (3H,s,H-Methylketon); 1,88 (3H,s,H-Propin); 0,65 (3H,s,H-18).

### Beispiel 18

### 17β-Hydroxy-17α-(3-hydroxyprop-1-inyl)-11β-[4-(4-methylthiophenyl)-phenyl]-4,6-estradien-3-on

a) 3-Ethoxy-17α-[3-(tetrahydropyran-2-yloxy)-prop-1-inyl]-11β-(4-trifluormethylsulfonyloxyphenyl)-3,5-estradien-17β-ol
   Aus 52,5 g 3-[(Tetrahydro-2*H*-pyran-2-yl)oxy]-1-propin in 1300 ml absolutem Tetrahydrofuran und 237 ml einer 1,6 molaren Lösung von Butyllithium in Hexan stellt man bei 0°C die lithiumorganische Verbindung her. Anschließend wird eine Lösung von 16,5 g der in Beispiel 11 d) hergestellten Verbindung in 300 ml absolutem Tetrahydrofuran hinzugetropft. Man läßt 1 h bei 0°C nachrühren, versetzt mit gesättigter Ammoniumchloridlösung und extrahiert mit Ethylacetat. Die organische Phase wird mit gesättigter Natriumchloridlösung gewaschen und über Natriumsulfat getrocknet. Es wird im Vakuum eingeengt. Das Rohprodukt wird durch eine Säulenchromatographie an Kieselgel mit einem Gemisch aus Hexan und Ethylacetat gereinigt. Man erhält 13,4 g der Titelverbindung als weißen Schaum.
b) 6β-Brom-17β-hydroxy-17α-[3-(tetrahydropyran-2-yloxy)-prop-1-inyl]-11β-(4-trifluormethylsulfonyloxyphenyl)-4-estren-3-on
   Analog der unter Beispiel 1 g) beschriebenen Vorschrift werden aus 13 g 3-Ethoxy-17α-[3-(tetrahydropyran-2-yloxy)-prop-1-inyl]-11β-(4-trifluormethylsulfonyloxyphenyl)-3,5-estradien-17β-ol 15,7 g der Titelverbindung als Rohprodukt erhalten.
c) 17β-Hydroxy-17α-[3-(tetrahydropyran-2-yloxy)-prop-1-inyl]-11β-(4-trifluormethylsulfonyloxyphenyl)-4,6-estradien-3-on
   Analog der unter Beispiel 1 h) beschriebenen Vorschrift werden aus 15,7 g 6β-Brom-17β-hydroxy-17α-[3-(tetrahydropyran-2-yloxy)-prop-1-inyl]-11β-(4-trifluormethylsulfonyloxyphenyl)-4-estren-3-on 8,7 g der Titelverbindung als gelblicher Schaum erhalten.
   ¹H-NMR (CDCl₃) δ [ppm]: 7,5 (2H,d J=9Hz,H-aromatisch); 7,21 (2H,d J=9Hz,H-aromatisch); 6,2-6,35 (2H,m,H-6 und H-7); 5,82 (1H,s,H-4); 4,83 (1H,m,H-Acetal); 4,3-4,38 (2H,m,H-22); 3,8-3,91 (1H,m,H-THP-Ether); 3,4-3,59 (2H,m,H-11 und H-THP-Ether); 0,58 (3H,s,H-18).
d) 17β-Hydroxy-11β-[4-(4-methylthiophenyl)-phenyl]-17α-[3-(tetrahydro-pyran-2-yloxy)--prop-1-inyl]-4,6-estradien-3-on
   Analog der unter Beispiel 6 a) beschriebenen Vorschrift werden aus 1,3 g 17β-Hydroxy-17α-[3-(tetrahydropyran-2-yloxy)-prop-1-inyl]-11β-(4-trifluormethylsulfonyloxyphenyl)--4,6-estradien-3-on durch Kupplung mit 4-Methylthiophenylboronsäure 1,05 g der Titelverbindung als gelblicher Schaum erhalten.
e) 17β-Hydroxy-17α-(3-hydroxyprop-1-inyl)-11β-[4-(4-methylthiophenyl)-phenyl]-4,6-estradien-3-on
   Analog der unter Beispiel 4 h) beschriebenen Vorschrift werden aus 1 g 17β-Hydroxy-11β-[4-(4-methylthiophenyl)-phenyl]-17α-[3-tetrahydropyran-2-yloxy)-prop-1-inyl]-4,6--estradien-3-on 690 mg der Titelverbindung als gelblicher Schaum erhalten.
   ¹H-NMR (CDCl₃) δ [ppm]: 7,42-7,57 (6H,m,H-aromatisch); 7,32 (2H,d J=9Hz,H-aromatisch); 6,22-6,34 (2H,m,H-6 und H-7); 5,81 (1H,s,H-4); 4,32-4,38 (2H,m,H-22); 3,48 (1H,tr breit J=6Hz,H-11); 2,52 (3H,s,H-Methylthioether); 0,66 (3H,s,H-18).

### Beispiel 19

### 17β-Hydroxy-17α-(3-hydroxyprop-1(Z)-enyl)-11β-[4-(4-methylthiophenyl)-phenyl]-4,6-estradien-3-on

Analog der unter Beispiel 4 i) beschriebenen Vorschritt werden aus 400 mg 17β-Hydroxy-17α-(3-hydroxyprop-1-inyl)-11β-[4-(4-methylthiophenyl)-phenyl]-4,6-estradien-3-on 214 mg der Titelverbindung als gelblicher Schaum erhalten.
¹H-NMR (CDCl₃) δ [ppm]: 7,42-7,56 (6H,m,H-aromatisch); 7,32 (2H,d J=9Hz,H-aromatisch); 6,22-6,33 (2H,m,H-6 und H-7); 5,6-5,88 (3H,m,H-4,H-20 und H-21); 4,23-4,32 (2H,m,H-22); 3,42 (1H,tr breit J=6Hz,H-11); 2,53 (3H,s,H-Methylthioether); 0,72 (3H,s,H-18).

### Beispiel 20

### 17α-Cyanmethyl-17β-hydroxy-11β-(4-hydroxyphenyl)-4,6-estradien-3-on

a) 17α-Cyanmethyl-3-ethoxy-11β-(4-hydroxyphenyl)-3,5-estradien-17β-ol
   Unter Schutzgas werden 29,9 ml Diisopropylamin in 440 ml absolutem Tetrahydrofuran vorgelegt und bei -30 °C mit 133 ml einer 1,6 m n-Butyllithiumlösung (Hexan) versetzt. Zur vollständigen Deprotonierung wird die Lösung bei 0 °C 30 Minuten nachgerührt, bevor sie auf -70 °C heruntergekühlt wird. Nach Zutropfen von 11,2 ml Acetonitril wird 1 Stunde bei -70 °C nachgerührt. Anschließend werden 13 g (Rohprodukt) der in Beispiel 11 d) hergestellten Verbindung, gelöst in 65 ml absolutem Tetrahydrofuran langsam zugetropft. Nach 60-minütigem Nachrühren wird das Reaktionsgemisch auf gesättigte Natriumhydrogencarbonatlösung gegossen und die wäßrige Phase mit Essigester extrahiert. Die vereinigten organischen Phasen werden mit gesättigter Natriumchloridlösung gewaschen, über Natriumsulfat getrocknet und am Va- kuum eingeengt. Der Rückstand wird an Kieselgel mit einem Gemisch aus Etylacetat/ Hexan chromatograpiert. Man erhält 11,5 g der Titelverbindung als weißen Schaum.
b) 6β-Brom-17α-cyanmethyl-17β-hydroxy-11β-(4-hydroxyphenyl)-4-estren-3-on
   Analog der unter Beispiel 1 g) beschriebenen Vorschrift werden aus 11 g 17α-Cyanmethyl-3-ethoxy-11β-(4-hydroxyphenyl)-3,5-estradien-17β-ol 5,9 g der Titelverbindung als Rohprodukt erhalten.
c) 17α-Cyanmethyl-17β-hydroxy-11β-(4-hydroxyphenyl)-4,6-estradien-3-on
   Analog der unter Beispiel 1 h) beschriebenen Vorschrift werden aus 5,7 g 6β-Brom-17α-cyanmethyl-17β-hydroxy-11β-(4-hydroxyphenyl)-4-estren-3-on 4,9 g der Titelverbindung als gelblicher Schaum erhalten.
   ¹H-NMR (CDCl₃) δ [ppm]: 7,17 (2H,d J=9Hz,H-aromatisch); 6,76 (2H,d J=9Hz,H-aromatisch); 6,21-6,3 (2H,m,H-6 und H-7); 5,75 (1H,s,H-4); 3,34 (1H,tr breit J=6Hz,H-11); 0,67 (3H,s,H-18)

### Beispiel 21

### 11β-(4-Acetylphenyl)-17α-cyanmethyl-17β-hydroxy-4,6-estradien-3-on

a) 17α-Cyanmethyl-17β-hydroxy-11β-(4-trifluormethylsulfonyloxyphenyl)-4,6-estradien-3-on
   Analog der unter Beispiel 4 d) beschriebenen Vorschrift werden aus 3 g 17α-Cyanmethyl-17β-hydroxy-11β-(4-hydroxyphenyl)-4,6-estradien-3-on 2,2 g der Titelverbindung als gelblicher Schaum erhalten.
   ¹H-NMR (CDCl₃) δ [ppm]: 7,52 (2H,d J=9Hz,H-aromatisch); 7,23 (2H,d J=9Hz,H-aromatisch); 6,17-6,35 (2H,m,H-6 und H-7); 5,83 (1H,s,H-4); 3,51 (1H,tr breit J=6Hz,H-11); 2,65 (1H,d J=17,5Hz,H-20); 2,52 (1H,d J=17,5Hz,H-20); 0,65 (3H,s,H-18)
b) 11β-(4-Acetylphenyl)-17α-cyanmethyl-17β-hydroxy-4,6-estradien-3-on
   Analog der unter Beispiel 4 f) und h) beschriebenen Vorschriften werden aus 400 mg 17α-Cyanmethyl-17β-hydroxy-11β-(4-trifluormethylsulfonyloxyphenyl)-4,6-estradien-3-on 230 mg der Titelverbindung als gelblicher Schaum erhalten.
   ¹H-NMR (CDCl₃) δ [ppm]: 7,89 (2H,d J=9Hz,H-aromatisch); 7,51 (2H,d J=9Hz,H-aromatisch); 6,2-6,34 (2H,m,H-6 und H-7); 5,82 (1H,s,H-4); 3,5 (1H,tr breit J=6Hz,H-11); 2,59 (3H,s,H-Methylketon); 2,69 (1H,d J=17,5Hz,H-20); 2,55 (1H,d J=17,5Hz,H-20); 0,71 (3H,s,H-18)
   Fp. = 293 °C (Zersetzung) (Ethylacetat)
   [α]²⁰_{D} = 153° (CHCl₃;c=0,54)

### Beispiel 22

### 17α-Cyanmethyl-17β-hydroxy-11β-[4-(2-propenyl)-phenyl]-4,6-estradien-3-on

Analog der unter Beispiel 4 f) beschriebenen Vorschrift werden aus 400 mg 17α-Cyanmethyl-17β-hydroxy-11β-(4-trifluormethylsulfonyloxyphenyl)-4,6-estradien-3-on durch Kupplung mit Allyl-tri-n-butylzinn 224 mg der Titelverbindung als gelblicher Schaum erhalten.
¹H-NMR (CDCl₃) δ [ppm]: 7,32 (2H,d J=9Hz,H-aromatisch); 7,1 (2H,d J=9Hz,H-aromatisch); 6,18-6,33 (2H,m,H-6 und H-7); 5,9-6,05 (1H,m,H-olefinisch); 5,8 (1H,s,H-4); 5,1 (1H,tr J=0,5Hz,H-olefinisch); 5,03-5,07 (1H,m,H-olefinisch); 3,44 (1H,tr breit J=6Hz,H-11); 3,37 (2H,d J=7,5Hz,H-benzylisch); 2,65 (1H,d J=17,5Hz,H-20); 2,5 (1H,d J=17,5Hz,H-20); 0,69 (3H,s,H-18)
Fp. = 248 °C (Zersetzung)
[α]²⁰_{D} = 92 ° (CHCl₃;c=0,52)

### Beispiel 23

### 17α-Cyanmethyl-17β-hydroxy-11β-[4-(4-methylthiophenyl)-phenyl]-4,6-estradien-3-on

Analog der unter Beispiel 6 a) beschriebenen Vorschrift werden aus 400 mg 17α-Cyanmethyl-17β-hydroxy-11β-(4-trifluormethylsulfonyloxyphenyl)-4,6-estradien-3-on durch Kupplung mit 4-Methylthiophenylboronsäure 356 mg der Titelverbindung als gelblicher Schaum erhalten.
¹H-NMR (CDCl₃) δ [ppm]: 7,43-7,57 (6H,m,H-aromatisch); 7,33 (2H,d J=9Hz,H-aromatisch); 6,19-6,35 (2H,m,H-6 und H-7); 5,81 (1H,s,H-4); 3,5 (1H,tr breit J=6Hz,H-11); 2,67 (1H,d J=17,5Hz,H-20); 2,53 (3H,s,H-Methylthioether); 2,52 (1H,d J=17,5Hz,H-20); 0,71 (3H,s,H-18)
Fp. = 186 °C
[α]²⁰_{D} = 209 ° (CHCl₃;c=0,505)

## Patentansprüche

1. Verbindungen der allgemeinen Formel I 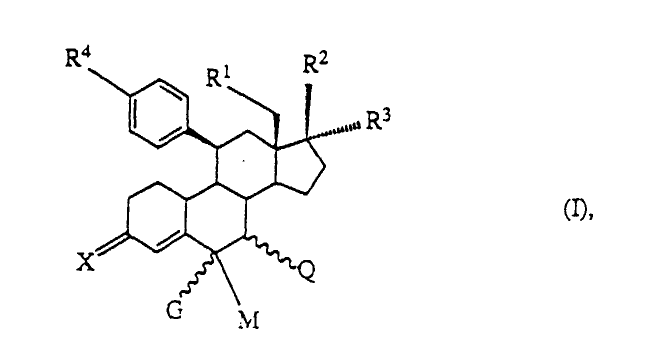 worin
X für ein Sauerstoffatom oder die Hydroxyiminogruppierung >N∼OH
R¹ für ein Wasserstoffatom oder eine Methylgruppe,
R² für eine Hydroxygruppe, eine C₁-C₁₀-Alkoxy- oder C₁-C₁₀-Acyloxygruppe,
R³ für ein Wasserstoffatom, die Gruppierung -(CH₂)ₙCH₂Z, wobei n 0, 1, 2, 3, 4 oder 5 ist, Z ein Wasserstoffatom, die Cyanogruppe oder den Rest -OR⁵ mit R⁵=H, C₁-C₁₀-Alkyl oder C₁-C₁₀-Acyl bedeuten, die Gruppierung -(CH₂)ₘC≡C-Y, wobei m 0, 1 oder 2 und Y ein Wasserstoff-, Fluor-, Chlor- Brom- oder Jod-Atom, einen C₁-C₁₀-Alkyl-, C₁-C₁₀-Hydroxyalkyl-, C₁-C₁₀-Alkoxyalkyl-, C₁-C₁₀-Acyloxyalkylrest bedeuten, die Gruppierung -(CH₂)ₚ-CH=CH-(CH₂)ₖCH₂R⁶, wobei p 0 oder 1 und k 0, 1 oder 2 und R⁶ ein Wasserstoffatom, eine Hydroxygruppe, einen C₁-C₄-Alkoxy- oder C₁-C₄-Acyloxyrest bedeuten,
oder aber R² und R³ gemeinsam für einen Rest der Formel 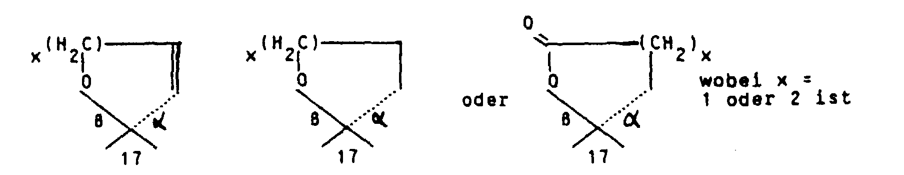
R⁴ für ein Wasserstoffatom, eine Cyanogruppe, ein Chlor-, Fluor-, Brom-, Jodatom, für eine Trialkylsilyl-, Trialkylstannylgruppe, für einen geradkettigen oder verzweigten, gesättigten oder ungesättigten C₁-C₈-Kohlenwasserstoff-Acyl- oder Alkoxyalkylrest, für
eine Aminogruppe 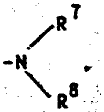 in welcher R⁷ und R⁸ unabhängig voneinander ein Wasserstoffatom oder eine C₁-C₄-Alkylgruppe bedeuten, oder für ein entsprechendes Aminoxid 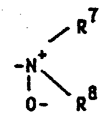 oder für die Gruppierungen -OR⁹ oder -S(O)ᵢR⁹ mit i = 0, 1 oder 2, in welchen R⁹ ein Wasserstoffatom, eine Methyl-, Ethyl-, Propyl-, Isopropyl-, Methoxyphenyl-, Allyl- oder eine 2-Dimethylaminoethylgruppe bedeuten, oder für einen Heteroarylrest der Formel Iα 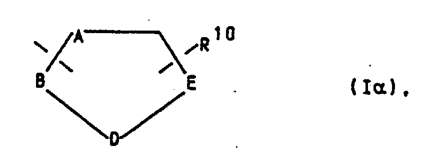 in welchem A ein Stickstoff-. Sauerstoff- oder Schwefelatom, -B-D-E- die Elementenfolge -C-C-C-, -N-C-C- oder -C-N-C- und R¹⁰ ein Wasserstoffatom, eine Cyanogruppe, ein Chlor-, Fluor-, Brom-, Jodatom, eine Trialkylsilyl-, Trialkylstannylgruppe, einen geradkettigen oder verzweigten, gesättigten oder ungesättigten C₁-C₈-Alkyl-, -Acyl- oder Alkoxyalkylrest, für
eine Aminogruppe 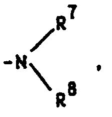 in welcher R⁷ und R⁸ unabhängig voneinander ein Wasserstoffatom oder eine C₁-C₄-Alkylgruppe bedeuten, oder ein entsprechendes Aminoxid 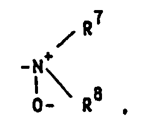 oder die Gruppierung -OR⁹ oder -S(O)ᵢR⁹ mit i = 0, 1 oder 2, in welchen R⁹ ein Wasserstoffatom, eine Methyl-, Ethyl-, Propyl-, Isopropyl-, Methoxyphenyl-, Allyl- oder eine 2-Dimethylaminoethylgruppe bedeuten, symbolisieren,
oder für einen Heteroarylrest der Formel Iβ 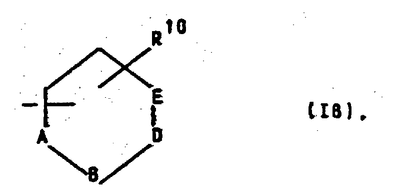 in welchem A ein Stickstoffatom und -B-D-E- die Elementenfolge -C-C-C-, -N-C-C-, -C-N-C- oder -C-C-N- bedeuten und R¹⁰ die bereits angegebene Bedeutung hat,
oder für einen Phenylrest der Formel Iγ 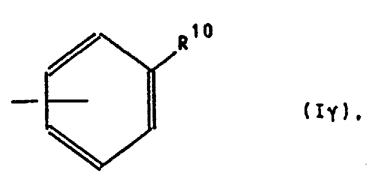 worin R¹⁰ die bereits angegebene Bedeutung hat,
G, wenn M und Q Wasserstoffatome darstellen, für ein Halogenatom oder einen C₁-C₄-Alkylrest oder wenn M und Q eine gemeinsame zusätzliche Bindung darstellen, für ein Wasserstoff-, ein Halogenatom oder einen C₁-C₄-Alkylrest, Q, wenn M und G Wasserstoffatome darstellen, für einen C₁-C₄-Alkylrest, wobei, wenn M und Q oder M und G Wasserstoffatome darstellen, R⁴ nicht für eine Acylgruppe stehen kann, G und M gemeinsam, wenn Q ein Wasserstoffatom darstellt, für eine Methylen- oder Ethylengruppe,
stehen.

2. Verbindungen nach Anspruch 1, nämlich
17β-Hydroxy-11β-(4-methoxyphenyl)-17α-(prop-1-inyl)-4,6-estradien-3-on
17β-Hydroxy-11β-(4-methoxyphenyl)-7β-methyl-17α-(prop-1-inyl)-4-estren-3-on
17β-Hydroxy-11β-(4-methoxyphenyl)-7α-methyl-17α-(prop-1-inyl)-4-estren-3-on
17β-Hydroxy-17α-(3-hydroxyprop-1-(Z)-enyl)-6β-methyl-11β-[4-(3-pyridinyl)-phenyl]-4-estren-3-on
17β-Hydroxy-17α-(3-hydroxyprop-1-(Z)-enyl)-6α-methyl-11β-[4-(3-pyridinyl)-phenyl]-4-estren-3-on
17β-Hydroxy-17α-(3-hydroxyprop-1-inyl)-6β-methyl-11β-[4-(3-pyridinyl)-phenyl]-4-estren-3-on
11β-(4-Dimethylaminophenyl)-17β-hydroxy-17α-(prop-1-inyl)-4,6-estradien-3-on
11β-(4-Dimethylaminophenyl)-17α-ethinyl-17β-hydroxy-4,6-estradien-3-on
11β-[4-(4-Cyanphenyl)-phenyl]-17β-hydroxy-17α-(prop-1-inyl)-4,6-estradien-3-on
11β-[4-(4-Cyanphenyl)-phenyl]-17β-hydroxy-17α-(3-hydroxyprop-1-inyl)-4,6-estradien-3-on
11β-[4-(4-Cyanphenyl)-phenyl]-17β-hydroxy-17α-(3-hydroxyprop-1(Z)-enyl)-4,6-estradien-3-on
17β-Hydroxy-11β-[4-(4-methylthiophenyl)-phenyl)-17α-(prop-1-inyl)-4,6-estradien-3-on
17β-Hydroxy-11β-[4-(4-methylsulfinylphenyl)-phenyl]-17α-(prop-1-inyl)-4,6-estradien-3-on
17β-Hydroxy-11β-[4-(4-methylsulfonylphenyl)-phenyl]-17α-(prop-1-inyl)-4,6-estradien-3-on
11β-[4-(4-Acetylphenyl)-phenyl)-17β-hydroxy-17α-(3-hydroxyprop-1(Z)-enyl)-4,6-estradien-3-on
17β-Hydroxy-11β-[4-(3-furanyl)-phenyl]-6β-methyl-17α-(3-hydroxyprop-1(Z)-enyl-4-estren-3-on
17β-Hydroxy-11β-[4-(3-furanyl)-phenyl]-6α-methyl-17α-(3-hydroxyprop-1(Z)-enyl-4-estren-3-on
17β-Hydroxy-6β-methyl-17α-(3-hydroxyprop-1(Z)-enyl)-11β-[4-(3-thienyl)-phenyl]-4-estren-3-on
17β-Hydroxy-6α-methyl-17α-(3-hydroxyprop-1(Z)-enyl)-11β-[4-(3-thienyl)-phenyl]-4-estren-3-on
17β-Hydroxy-6β-methyl-17α-(3-hydroxyprop-1(Z)-enyl)-11β-[4-(2-thiazolyl)-phenyl]-4-estren-3-on
17β-Hydroxy-6α-methyl-17α-(3-hydroxyprop-1(Z)-enyl)-11β-[4-(2-thiazolyl)-phenyl]-4-estren-3-on
17β-Hydroxy-11β-[4-(5-pyrimidinyl)-phenyl)-6β-methyl-17α-(3-hydroxyprop-1(Z)-enyl)-4-estren-3-on
17β-Hydroxy-11β-[4-(5-pyrimidinyl)-phenyl]-6α-methyl-17α-(3-hydroxyprop-1(Z)-enyl)-4-estren-3-on
17β-Hydroxy-11β-[4-(5-pyrimidinyl)-phenyl]-6β-methyl-17α-(prop-1-inyl)-4-estren-3-on

3. Zwischenprodukte der allgemeinen Formel IX 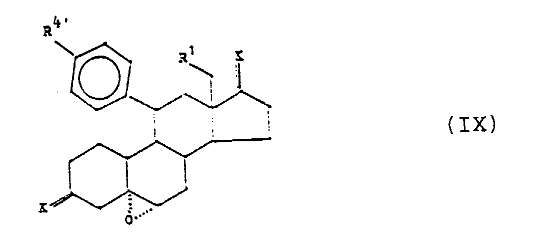 worin
R¹ für ein Wasserstoff oder eine Methylgruppe
R^{4'} für einen der Reste R⁴ in der allgemeinen Formel I und
K für 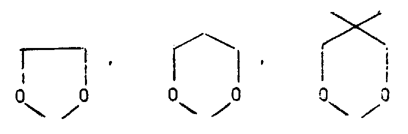 oder ein anderes Sauerstoffketal
stehen.

4. Verfahren zur Herstellung von Verbindungen der allgemeinen Formel I 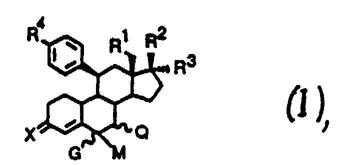 worin die Substituenten R¹, R², R³, R⁴, X, G, M und Q die in Anspruch 1 angegebene Bedeutung haben, dadurch gekennzeichnet, daß entweder
a) eine Verbindung der allgemeinen Formel VIII 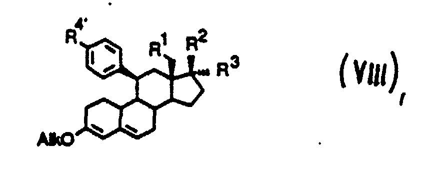 worin R¹, R² und R³ die in Formel I angegebene Bedeutung haben, R^{4'} entweder dieselbe Bedeutung wie R⁴ in Formel I hat oder für eine Perfluoralkylsulfonyloxygruppe CₙF₂ₙ₊₁SO₂O- (n = 1, 2, 3, 4) steht sowie Alk ein C₁-C₄-Alkylrest ist, durch Bromierung und nachfolgende Dehydrobromierung im alkalischen Medium und anschließende saure Behandlung in eine Verbindung I mit 3-Keto-4,6-dien-Teilstruktur überführt und danach gegebenenfalls ein C₁-C₄-Alkylrest in 7-Position eingeführt oder
b) eine Verbindung der allgemeinen Formel XIII 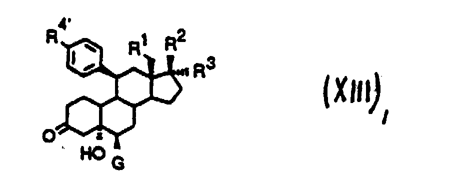 worin R^{1,} R² und R³ die in Formel I angegebene Bedeutung haben, R^{4'} entweder dieselbe Bedeutung wie R⁴ in Formel I hat oder für eine Perfluoralkylsulfonyloxygruppe CₙF₂ₙ₊₁SO₂O- (n = 1, 2, 3, 4) steht sowie G ein C₁-C₄-Alkylrest oder ein Halogenatom ist, durch basische Behandlung in eine Verbindung I mit 3-Keto-4-en-6-alkyl- bzw. halogen-Teilstruktur überführt und danach gegebenenfalls eine 6β-Alkylgruppe epimerisiert und wenn R^{4'}in der Verbindung der allgemeinen Formel VIII bzw. XIII für eine Perfluoralkylsulfonyloxygruppe CₙF₂ₙ₊₁SO₂O- steht, nach Ausführung des Reaktionsschrittes a) oder b) die entsprechende Perfluoralkylsulfonyloxy-Verbindung mit 3-Keto-4,6-dien- oder 3-Keto-4-en-6-alkyl- bzw. halogen-Teilstruktur
c) entweder mit einer Verbindung der allgemeinen Formel W
R^{4"} - M (W),
worin M für einen der Reste 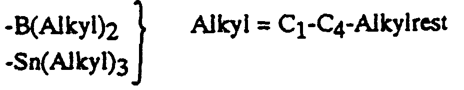 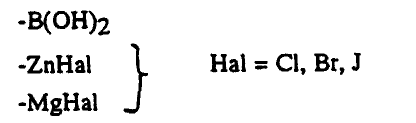 steht und R^{4"} dieselbe Bedeutung wie R⁴ in Formel I hat oder einen geschützten Vorläufer von R⁴ bedeutet, umgesetzt oder
d) intermediär und übergangsmetallkatalysiert in eine entsprechende Tri-organylstannyl-, vorzugsweise Tri-n-(C₁-C₄-alkylstannyl)-Verbindung überführt und diese anschließend mit einer Halogenverbindung Y
R⁴ - Hal (Y),
worin R⁴ die in Formel I angegebene Bedeutung hat und Hal für ein Brom- oder Jodatom steht, umgesetzt oder
wenn R^{4'} in der Verbindung der allgemeinen Formel VIII bzw. Formel XIII für eine Perfluoralkylsulfonyloxygruppe CₙF₂ₙ₊₁SO₂O- steht,
zuerst der Reaktionsschritt c) oder d) und anschließend der Reaktionsschritt a) oder b) ausgeführt, gegebenenfalls vorhandene Schutzgruppen abgespalten und anschließend gewünschtenfalls in R² und/oder R³ und/oder R⁴ vorhandene Hydroxy-, Mercapto- und/oder Aminogruppe(n) alkyliert bzw. acyliert, gewünschtenfalls ein Cyanidrest in den 11β-Arylsubstituenten eingeführt, gewünschtenfalls die m R⁴ gegebenenfalls enthaltene Amino- oder Sulfidgruppe oxidiert, gewünschtenfalls mit Hydroxylamin-hydrochlorid zum Produkt der allgemeinen Formel I mit X in der Bedeutung der Hydroxyiminogruppierung >N∼OH umgesetzt oder die 3-Oxogruppe in ein Produkt der allgemeinen Formel I, worin X für 2 Wasserstoffatome steht, umgewandelt sowie gegebenenfalls ein pharmazeutisch verträgliches Säureadditionssalz hergestellt werden/wird.

5. Pharmazeutische Präparate, die mindestens eine Verbindung gemäß Anspruch 1 oder 2 sowie einen pharmazeutischen Träger enthalten.

6. Verwendung der Verbindungen gemäß Anspruch 1 oder 2 zur Herstellung von Arzneimitteln.

## Claims

1. Compounds of the general formula I 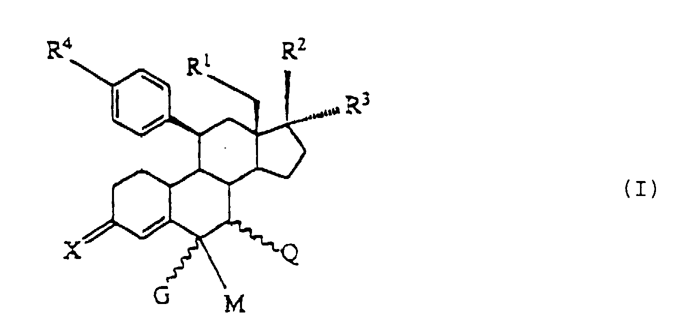 in which
X represents an oxygen atom or the hydroxyimino grouping >N∼OH,
R¹ represents a hydrogen atom or a methyl group.
R² represents a hydroxy group, a C₁-C₁₀-alkoxy group or a C₁-C₁₀-acyloxy group,
R³ represents a hydrogen atom; the grouping -(CH₂)ₙCH₂Z wherein n is 0, 1, 2, 3, 4 or 5 and Z represents a hydrogen atom, a cyano group or the radical -OR⁵ in which R⁵ = H, C₁-C₁₀-alkyl or C₁-C₁₀-acyl; the grouping -(CH₂)ₘC≡C-Y wherein m is 0, 1 or 2 and Y represents a hydrogen, fluorine, chlorine, bromine or iodine atom, or a C₁-C₁₀-alkyl-, C₁-C₁₀-hydroxyalkyl, C₁-C₁₀-alkoxyalkyl or C₁-C₁₀-acyloxyalkyl radical; or the grouping -(CH₂)ₚ-CH=CH-(CH₂)ₖCH₂R⁶ wherein p is 0 or 1 and k is 0, 1 or 2 and R⁶ represents a hydrogen atom, a hydroxy group, a C₁-C₄-alkoxy radical or a C₁-C₄-acyloxy radical,
or alternatively R² and R³ together represent a radical of the formula 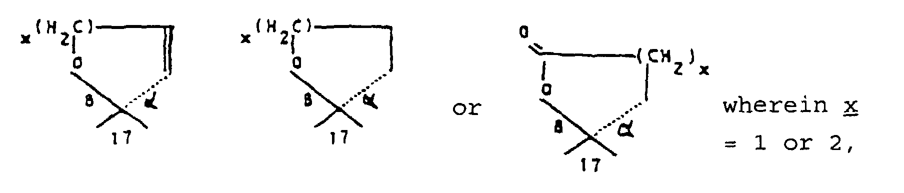 R⁴ represents a hydrogen atom; a cyano group; a chlorine, fluorine, bromine or iodine atom; a trialkylsilyl group; a trialkylstannyl group; a straight-chain or branched, saturated or unsaturated, C₁-C₈-hydrocarbon, -acyl or alkoxyalkyl radical; an
amino group 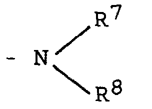 in which R⁷ and R⁸, each independently of the other, represents a hydrogen atom or a C₁-C₄-alkyl group; a corresponding amine oxide 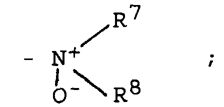 or the grouping -OR⁹ or -S(O)ᵢR⁹ in which i = 0, 1 or 2 and R⁹ represents a hydrogen atom, a methyl, ethyl, propyl, isopropyl, methoxyphenyl, allyl or 2-dimethylaminoethyl group; or R⁴ represents a heteroaryl radical of formula Iα 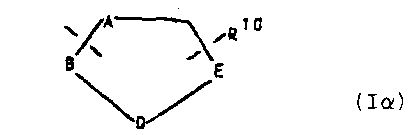 in which A represents a nitrogen, oxygen or sulphur atom, -B-D-E- represents the sequence of elements -C-C-C-, -N-C-C- or -C-N-C- and R¹⁰ represents a hydrogen atom; a cyano group; a chlorine, fluorine, bromine or iodine atom; a trialkylsilyl group; a trialkylstannyl group; a straight-chain or branched, saturated or unsaturated C₁-C₈-alkyl, -acyl or alkoxyalkyl radical; an
amino group 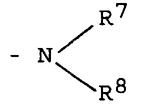 in which R⁷ and R⁸, each independently of the other, represents a hydrogen atom or a C₁-C₄-alkyl group; a corresponding amine oxide 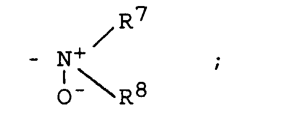 or the grouping -OR⁹ or -S(O)ᵢR⁹ in which i = 0, 1 or 2 and R⁹ represents a hydrogen atom, a methyl, ethyl, propyl, isopropyl, methoxyphenyl, allyl or 2-dimethylaminoethyl group;
or R⁴ represents a heteroaryl radical of formula Iβ 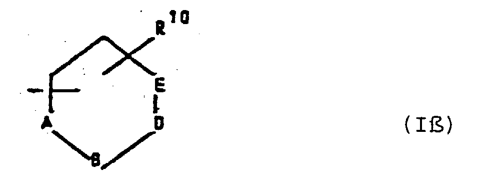 in which A represents a nitrogen atom and -B-D-E- represents the sequence of elements -C-C-C-, -N-C-C-, -C-N-C- or -C-C-N- and R¹⁰ has the meaning already given,
or R⁴ represents a phenyl radical of formula Iγ 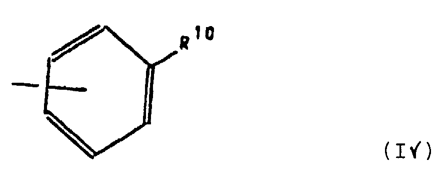 in which R¹⁰ has the meaning already given, and
G represents a halogen atom or a C₁-C₄-alkyl radical when M and Q are hydrogen atoms, or represents a hydrogen atom, a halogen atom or a C₁-C₄-alkyl radical when M and
Q together are an additional bond,
Q represents a C₁-C₄-alkyl radical when M and G are hydrogen atoms,
and when M and Q or M and G are hydrogen atoms, R⁴ may not represent an acyl group,
and G and M together represent a methylene or ethylene group when Q is a hydrogen atom.

2. Compounds according to claim 1, that is
17β-hydroxy-11β-(4-methoxyphenyl)-17α-(prop-1-ynyl)-4,6-oestradien-3-one
17β-hydroxy-11β-(4-methoxyphenyl)-7β-methyl-17α-(prop-1-ynyl)-4-oestren-3-one
17β-hydroxy-11β-(4-methoxyphenyl)-7α-methyl-17α-(prop-1-ynyl)-4-oestren-3-one
17β-hydroxy-17α-(3-hydroxyprop-1(Z)-enyl)-6β-methyl-11β-[4-(3-pyridyl)-phenyl]-4-oestren-3-one
17β-hydroxy-17α-(3-hydroxyprop-1(Z)-enyl)-6α-methyl-11β-[4-(3-pyridyl)-phenyl]-4-oestren-3-one
17β-hydroxy-17α-(3-hydroxyprop-1-ynyl)-6β-methyl-11β-[4-(3-pyridyl)-phenyl]-4-oestren-3-one
11β-(4-dimethylaminophenyl)-17β-hydroxy-17α'-(prop-1-ynyl)-4,6-oestradien-3-one
11β-(4-dimethylaminophenyl)-17α-ethynyl-17β-hydroxy-4,6-oestradien-3-one
11β-[4-(4-cyanophenyl)-phenyl]-17β-hydroxy-17α-(prop-1-ynyl)-4,6-oestradien-3-one
11β-[4-(4-cyanophenyl)-phenyl]-17β-hydroxy-17α-(3-hydroxyprop-1-ynyl)-4,6-oestradien-3-one
11β-[4-(4-cyanophenyl)-phenyl]-17β-hydroxy-17α-(3-hydroxyprop-1(Z)-enyl)-4,6-oestradien-3-one
17β-hydroxy-11β-[4-(4-methylthiophenyl)-phenyl]-17α-(prop-1-ynyl)-4,6-oestradien-3-one
17β-hydroxy-11β-[4-(4-methylsulphinylphenyl)-phenyl]-17α-(prop-1-ynyl)-4,6-oestradien-3-one
17β-hydroxy-11β-[4-(4-methylsulphonylphenyl)-phenyl]-17α-(prop-1-ynyl)-4,6-oestradien-3-one
11β-[4-(4-acetylphenyl)-phenyl]-17β-hydroxy-17α-(3-hydroxyprop-1(Z)-enyl)-4,6-oestradien-3-one
17β-hydroxy-11β-[4-(3-furanyl)-phenyl]-6β-methyl-17α-(3-hydroxyprop-1(Z)-enyl)-4-oestren-3-one
17β-hydroxy-11β-[4-(3-furanyl)-phenyl]-6α-methyl-17α-(3-hydroxyprop-1(Z)-enyl)-4-oestren-3-one
17β-hydroxy-6β-methyl-17α-(3-hydroxyprop-1(Z)-enyl)-11β-[4-(3-thienyl)-phenyl]-4-oestren-3-one
17β-hydroxy-6α-methyl-17α-(3-hydroxyprop-1(Z)-enyl)-11β-[4-(3-thienyl)-phenyl]-4-oestren-3-one
17β-hydroxy-6β-methyl-17α-(3-hydroxyprop-1(Z)-enyl)-11β-[4-(2-thiazolyl)-phenyl]-4-oestren-3-one
17β-hydroxy-6α-methyl-17α-(3-hydroxyprop-1(Z)-enyl)-11β-[4-(2-thiazolyl)-phenyl]-4-oestren-3-one
17β-hydroxy-11β-[4-(5-pyrimidinyl)-phenyl]-6β-methyl-17α-(3-hydroxyprop-1(Z)-enyl)-4-oestren-3-one
17β-hydroxy-11β-[4-(5-pyrimidinyl)-phenyl]-6α-methyl-17α-(3-hydroxyprop-1(Z)-enyl)-4-oestren-3-one
17β-hydroxy-11β-[4-(5-pyrimidinyl)-phenyl]-6β-methyl-17α-(prop-1-ynyl)-4-oestren-3-one

3. Intermediates of the general formula IX 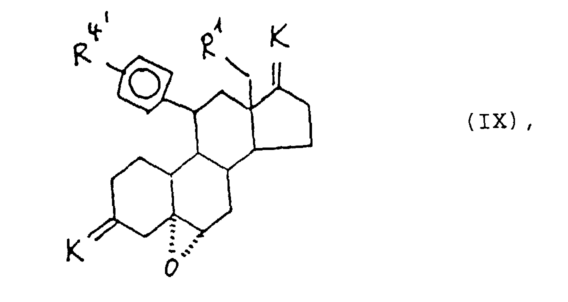 in which
R¹ represents a hydrogen atom or a methyl group
R^{4'} represents one of the radicals R⁴ in the general formula I and
K represents 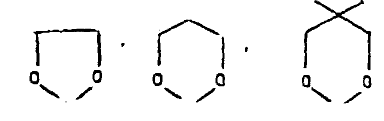 or another oxygen ketal.

4. A process for the preparation of compounds of the general formula I 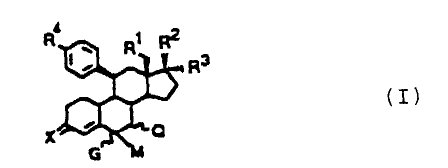 in which the substituents R¹, R², R³, R⁴, X, G, M and Q have the meanings given in claim 1, characterised in that either
a) a compound of the general formula VIII 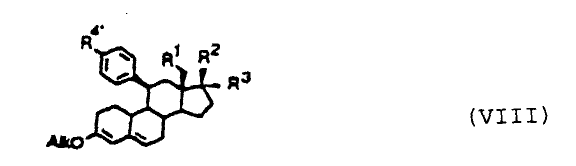 in which R¹, R² and R³ have the meanings given in formula I, R^{4'} either has the same meaning as R⁴ in formula I or represents a perfluoroalkylsulphonyloxy group cₙF₂ₙ₊₁SO₂O- (n = 1, 2, 3 or 4), and Alk represents a C₁-C₄-alkyl radical, is converted by bromination and subsequent dehydrobromination in alkaline medium, followed by treatment with acid, into a compound I having a 3-keto-4,6-diene partial structure and then, optionally, a C₁-C₄-alkyl radical is introduced into the 7-position, or
b) a compound of the general formula XIII 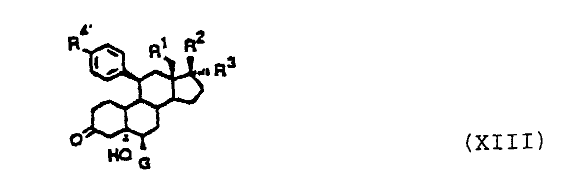 in which R¹, R² and R³ have the meanings given in formula I, R^{4'} either has the same meaning as R⁴ in formula I or represents a perfluoroalkylsulphonyloxy group CₙF₂ₙ₊₁SO₂O- (n = 1, 2, 3 or 4), and G represents a C₁-C₄-alkyl radical or a halogen atom, is converted by treatment with a base into a compound I having a 3-keto-4-en-6-alkyl or halogen partial structure and then, optionally, a 6β-alkyl group is epimerised and, if R^{4'} in the compound of the general formula VIII or XIII represents a perfluoroalkylsulphonyloxy group CₙF₂ₙ₊₁SO₂O-, after carrying out reaction step a) or b) the corresponding perfluoroalkylsulphonyloxy compound having a 3-keto-4,6-diene or 3-keto-4-en-6-yl or halogen partial structure is either
c) reacted with a compound of the general formula W
R^{4"} - M (W)
in which M represents one of the radicals 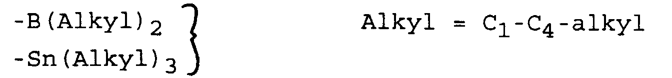 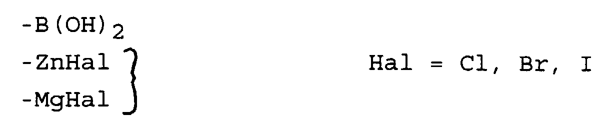 and R^{4"} has the same meaning as R⁴ in formula I or represents a protected precursor of R⁴, or
d) intermediately converted with transition metal catalysis into a corresponding tri-organylstannyl compound, preferably a tri-n-(C₁-C₄-alkylstannyl) compound, and this is then reacted with a halogen compound Y
R⁴-Hal (Y)
in which R⁴ has the meaning given in formula I and Hal represents a bromine or iodine atom or if R^{4'} in the compound of the general formula VIII or formula XIII represents a perfluoroalkylsulphonyloxy group CₙF₂ₙ₊₁SO₂O-,
first reaction step c) or d) and then reaction step a) or b) is carried out,
protecting groups that may be present are removed and then, if desired, hydroxy, mercapto and/or amino groups present in R² and/or R³ and/or R⁴ are alkylated or acylated, if desired a cyanide radical is introduced into the 11β-aryl substituent, if desired the amino or sulphide group that may be present in R⁴ is oxidised, if desired reaction is carried out with hydroxylamine hydrochloride to form the product of the general formula I in which X represents the hydroxyimino grouping 〉N∼OH, or the 3-oxo group is converted into a product of the general formula I in which X represents 2 hydrogen atoms and, if desired, a pharmaceutically tolerable acid addition salt is produced.

5. Pharmaceutical compositions that comprise at least one compound according to claim 1 or 2 as well as a pharmaceutical carrier.

6. The use of compounds according to claim 1 or 2 for the preparation of medicaments.

## Revendications

1. Composés de formule générale I 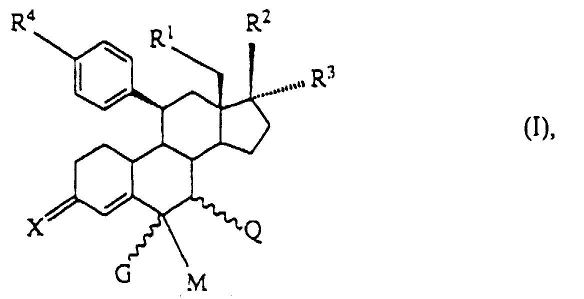 dans laquelle
X représente un atome d'oxygène ou le groupement hydroxyimino >N∼OH
R¹ représente un atome d'hydrogène ou un groupe méthyle,
R² représente un groupe hydroxy, un groupe alcoxy en C₁-C₁₀ ou un groupe acyloxy en C₁-C₁₀,
R³ représente un atome d'hydrogène, le groupement (CH₂)ₙCH₂Z , dans lequel n vaut 0, 1, 2, 3, 4 ou 5, Z représente un atome d'hydrogène, le groupe cyano ou le radical -OR⁵ avec R⁵ = H, alkyle en C₁-C₁₀ ou acyle en C₁-C₁₀, le groupement -(CH₂)ₘC≡C-Y , où m vaut 0, 1 ou 2 et Y représente un atome d'hydrogène, de fluor, de chlore, de brome ou d'iode, un radical alkyle en C₁-C₁₀, hydroxyalkyle en C₁-C₁₀, alcoxyalkyle en C₁-C₁₀ , acyloxyalkyle en C₁-C₁₀ , le groupement -(CH₂)_{P}-CH=CH-(CH₂)ₖCH₂R⁶, où p vaut 0 ou l et k vaut 0, 1 ou 2 et R⁶ représente un atome d'hydrogène, un groupe hydroxy, un radical alcoxy en C₁-C₄ ou un radical acyloxy en C₁-C₄,
ou encore R² et R³ représentent ensemble un radical de formule 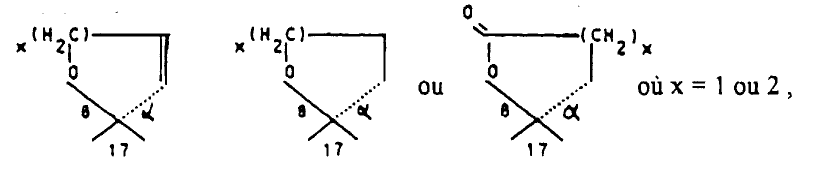
R⁴ représente un atome d'hydrogène, un groupe cyano, un atome de chlore, de fluor, de brome ou d'iode, un groupe trialkylsilyle, un groupe trialkylstannyle, un radical hydrocarboné, acyle ou alcoxyalkyle en C₁-C₈ à chaîne droite ou ramifiée, saturé ou insaturé, un groupe amino 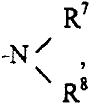 dans lequel R⁷ et R⁸ représentent indépendamment l'un de l'autre un atome d'hydrogène, ou un groupe alkyle en C₁-C₄, ou représentent un aminoxyde correspondant 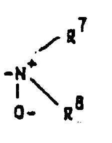 ou les groupements -OR⁹ ou -S(O)ᵢR⁹ avec i = 0, 1 ou 2, où R⁹ représente un atome d'hydrogène, un groupe méthyle, éthyle, propyle, isopropyle, méthoxyphényle, allyle ou 2-diméthylaminoéthyle, ou un radical hétéroaryle de formule Iα 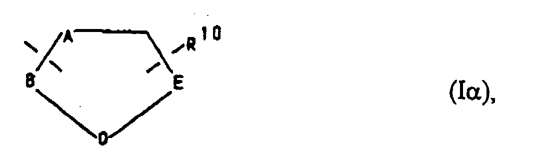 dans laquelle A représente un atome d'azote, d'oxygène ou de soufre, -B-D-E- représente la suite d'éléments -C-C-C-, -N-C-C- ou -C-N-C- et R¹⁰ représente un atome d'hydrogène, un groupe cyano, un atome de chlore, de fluor, de brome ou d'iode, un groupe trialkylsilyle, un groupe triallkylstannyle, un radical alkyle, acyle ou alcoxyalkyle en C₁-C₈ à chaîne droite ou ramifiée, saturé ou insaturé, un groupe amino 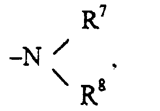 dans lequel R⁷ et R⁸ représentent indépendamment l'un de l'autre un atome d'hydrogène ou un groupe alkyle en C₁ C₄, ou un aminoxyde correspondant 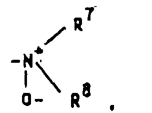 ou le groupement -OR⁹ ou -S(O)ᵢR⁹ avec i = 0, 1 ou 2 , dans lequel R⁹ représente un atome d'hydrogène, un groupe méthyle, éthyle, propyle, isopropyle, méthoxyphényle, allyle ou 2-diméthylaminoéthyle,
ou un radical hétéroaryle de formule Iβ 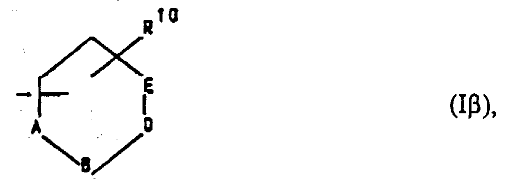 dans laquelle A représente un atome d'azote et -B-D-E- représentent la suite d'éléments -C-C-C-, -N-C-C- , -C-N-C- ou -C-C-N- et R¹⁰ a la signification déjà indiquée,
ou un radical phényle de formule Iγ 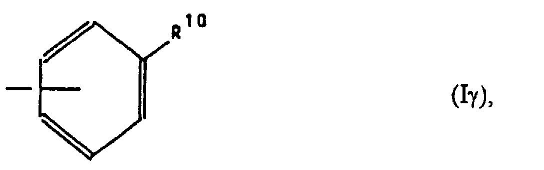 dans laquelle R¹⁰ a la signification déjà indiquée,
G, lorsque M et Q représentent des atomes d'hydrogène, symbolise un atome d'halogène ou un radical alkyle en C₁-C₄, ou, lorsque M et Q représentent une liaison supplémentaire commune, symbolise un atome d'hydrogène, un atome d'halogène ou un radical alkyle en C₁-C₄,
Q, lorsque M et G représentent des atomes d'hydrogène, symbolise un radical alkyle en C₁-C₄ , tandis que, lorsque M et Q ou M et G représentent des atomes d'hydrogène, R⁴ ne peut être un groupe acyle,
G et M ensemble, lorsque Q représente un atome d'hydrogène, symbolisent un groupe méthylène ou un éthylène.

2. Composés selon la revendication 1 à savoir
17β-hydroxy-11β-(4-méthoxyphényl)-17α-(prop-1-ynyl)-4,6-oestradiène-3-one,
17β-hydroxy-11β-(4-méthoxyphényl)-7β-méthyl-17α-(prop-1-ynyl)-4-oestrène-3-one,
17β-hydroxy-11β-(4-méthoxyphényl)-7α-méthyl-17α-(prop-1-ynyl)-4-oestrène-3-one,
17β-hydroxy-17α-(3-hydroxyprop-1-(Z)-ényl)-6β-méthyl-11β-[4-(3-pyridinyl)-phényl]-4-oestrène-3-one,
17β-hydroxy-17α-(3-hydroxyprop-1-(Z)-ényl)-6α-méthyl-11β-[4-(3-pyridinyl)-phényl]-4-oestrène-3-one,
17β-hydroxy-17α-(3-hydroxyprop-1-ynyl)-6β-méthyl-11β-[4-(3-pyridinyl)-phényl]-4-oestrène-3-one,
11β-(4-diméthylaminophényl))-17β-hydroxy-17α-(prop-1-ynyl)-4,6-oestradiène-3 -one,
11β-(4-diméthylaminophényl)-17α-éthynyl-17β-hydroxy-4,6-oestradiène-3-one,
11β-[4-(4-cyanophényl)-phényl]-17β-hydroxy-17α-(prop-1-ynyl)-4,6-oestradiène-3-one,
11β-[4-(4-cyanophényl)-phényl]-17β-hydroxy-17α-(3-hydroxyprop-1-ynyl)-4,6-oestradiène-3-one,
11β-[4-(4-cyanophényl)-phényl]-17β-hydroxy-17α-(3-hydroxyprop-1(Z)-ényl)4,6-oestradiène-3-one,
17β-hydroxy-11β-[4-(4-méthylthiophényl)-phényl]-17α-(prop-1-ynyl)-4,6-oestradiène-3-one,
17β-hydroxy-11β-[4-(4-méthylsulfinylphényl)-phényl]-17α-(prop-1-ynyl)-4,6-oestradiène-3-one,
17β-hydroxy-11β-[4-(4-méthylsulfonylphényl)-phényl]-17α-(prop-1-ynyl)-4,6-oestradiène-3-one,
11β-[4-(4-acétylphényl)-phényl]-17β-hydroxy-17α-(3-hydroxyprop-1(Z)-ényl)-4,6-oestradiène-3-one,
17β-hydroxy-11β-[4-(3-furanyl)-phényl]-6β-méthyl-17α-(3-hydroxyprop-1(Z)-ényl)-4-oestrène-3-one,
17β-hydroxy-11β-[4-(3-furanyl)-phényl]-6α-méthyl-17α-(3-hydroxyprop-1(Z)-ényl)-4-oestrène-3-one,
17β-bydroxy-6β-méthyl-17α-(3-hydroxyprop-1(Z)-ényl)-11β-[4-(3-thiényl)-phényl]-4-oestrène-3-one,
17β-hydroxy-6α-méthyl-17α-(3-hydroxyprop-1(Z)-ényl)-11β-[4-(3-thiényl)-phényl]-4-oestrène-3 -one,
17β-hydroxy-6β-méthyl-17α-(3-hydroxyprop-1(Z)-ényl)-11β-[4-(2-thiazolyl)-phényl]-4-oestrène-3-one,
17β-hydroxy-6α-méthyl-17α-(3-hydroxyprop-1(Z)-ényl)-11β-[4-(2-thiazolyl)-phényl]-4-oestrène-3-one,
17β-hydroxy-11β-[4-(5-pyrimidinyl)-phényl]-6β-méthyl-17α-(3-hydroxyprop-1(Z)-ényl)4-oestrène-3-one,
17β-hydroxy-11β-[4-(5-pyrimidinyl)-phényl]-6α-méthyl-17α-(3-hydroxyprop-1(Z)-ényl)-4-oestrène-3-one,
17β-hydroxy-11β-[4-(5-pyrimidinyl)-phényl]-6β-méthyl-17α-(prop-1-ynyl)-4-oestrène-3-one,

3. Produits intermédiaires de formule générale IX 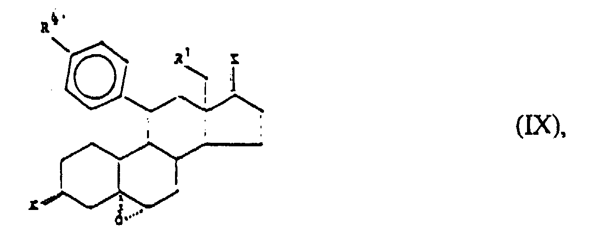 dans laquelle
R¹ représente un hydrogène ou un groupe méthyle,
R^{4'} représente l'un des radicaux R⁴ dans la formule générale I et
K représente 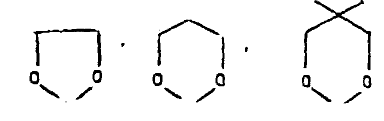 ou un autre cétal oxygéné.

4. Procédé de préparation de composés de formule générale I 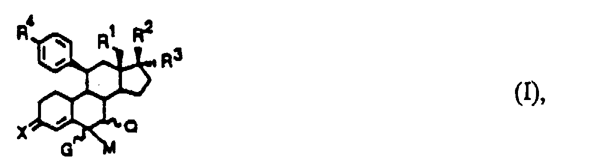 dans laquelle les substituants R¹, R², R³, R⁴, X, G, M et Q ont la signification indiquée dans la revendication 1,
caractérisé en ce que
a) ou bien l'on transforme un composé de formule générale VIII 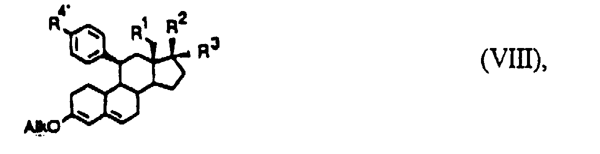 dans laquelle R¹, R² et R³ ont la signification indiquée dans la formule 1, R^{4'} ou bien a la même signification que R⁴ dans la formule I, ou bien représente un groupe perfluoroalkylsulfonyloxy CₙF₂ₙ₊₁SO₂O- (n=1,2,3,4) et Alk est un radical alkyle en C₁-C₄, par bromation puis déshydrobromation en milieu alcalin, et ensuite par traitement acide, en un composé I à structure partielle 3-céto-4,6-diéne, puis le cas échéant on introduit un radical alkyle en C₁-C₄ en position 7,
b) ou bien l'on transforme un composé de formule générale XIII 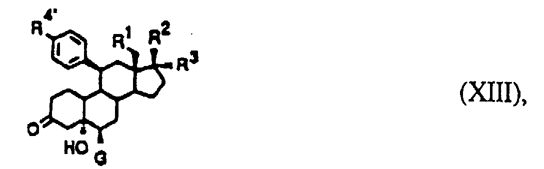 dans laquelle R¹, R² et R³ ont la signification indiquée dans la formule I, R^{4'} ou bien a la même signification que R⁴ dans la formule 1, ou bien représente un groupe perfluoroalkylsulfonyloxy CₙF₂ₙ₊₁SO₂O- (n=1,2,3,4) et G représente un radical alkyle en C₁-C₄ ou un atome d'halogène, par traitement basique, en un composé I à structure partielle 3-céto-4-ène-6-alkyle ou halogène, puis le cas échéant on épimérise un groupe 6β-alkyle et, lorsque R^{4'} dans le composé de formule générale VIII ou XIII représente un groupe perfluoroalkylsulfonyloxy CₙF₂ₙ₊₁SO₂O-, après réalisation des étapes de réaction a) ou b), on fait réagir le composé perfluoroalkylsulfonyloxy correspondant à structure partielle 3-céto-4,6-diène- ou 3-céto-4-ène-6-alkyle ou halogène
c) soit avec un composé de formule générale W
R^{4"} - M (W),
dans laquelle M représente l'un des radicaux 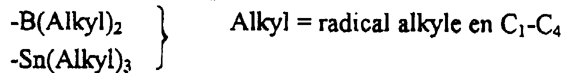 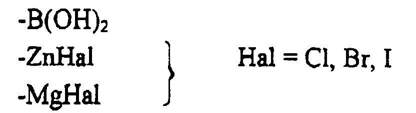 et R^{4"} a la même signification que R⁴ dans la formule I ou représente un précurseur protégé de R⁴, ou
d) soit on le transforme de façon intermédiaire et sous catalyse par des métaux de transition, en un composé tri-organylstannyle, de préférence tri-n-(alkyle en C₁-C₄-stannyle), puis on fait réagir celui-ci avec un composé halogéné Y
R⁴ - Hal (Y),
où R⁴ a la signification indiquée dans la formule I et Hal représente un atome de brome ou d'iode, ou
lorsque R^{4'} dans le composé de formule générale VIII ou XIII représente un groupe perfluoroalkylsulfonyloxy CₙF₂ₙ₊₁SO₂O-,
on effectue tout d'abord l'étape réactionnelle c) ou d) puis l'étape réactionnelle a) ou b), le cas échéant on sépare les groupes protecteurs présents puis si on le désire on alkyle ou selon les cas on acyle le ou les groupe(s) hydroxy, mercapto et/ou amino présents dans R² et/ou R³ et/ou R⁴, si on le désire on introduit un radical cyanure dans le substituant 11β-aryle, si on le désire on oxyde le groupe amino ou sulfure éventuellement contenu dans R⁴, si on le désire on le transforme avec du chlorhydrate d'hydroxylamine en un produit de formule générale I dans laquelle X représente un groupement hydroxyimino >N∼OH ou on transforme le groupe 3-oxo en un produit de formule générale I dans laquelle X représente 2 atomes d'hydrogène, et le cas échéant on prépare un sel d'addition d'acide pharmaceutiquement acceptable.

5. Préparations pharmaceutiques qui contiennent au moins un composé selon la revendication 1 ou 2 ainsi qu'un support pharmaceutique.

6. Utilisation des composés selon la revendication 1 ou 2 pour la production de médicaments.
